# EUROPEAN PATENT APPLICATION

(11) **EP 2 100 573 A1**
(43) Date of publication of application: **16.09.2009**
(21) Application number: 07850937.9
(22) Date of filing: 20.12.2007
(51) Int. Cl.: A61F 2/46, A61B 17/56

(54) **ARTIFICIAL JOINT REPLACEMENT ASSISTING DEVICE, ARTIFICIAL JOINT REPLACEMENT ASSISTING METHOD USING SAME, AND ASSISTING SYSTEM**

(30) Priority: 25.12.2006 JP 2006347017
(71) Applicant: Softcube Co., Ltd., Moriguchi-shi, Osaka 570-0083 (JP); Tatsumi, Ichiro, Osaka-shi, Osaka 545-0053 (JP)
(72) Inventor: TATSUMI, Ichiro, Osaka-shi Osaka 545-0053 (JP); HIRAKAWA, Kazuo, Fujisawa-shi Kanagawa 252-0804 (JP); KITAMURA, Yugo, Moriguchi-shi Osaka 570-0083 (JP); NAKAJIMA, Shinichi, Moriguchi-shi Osaka 570-0083 (JP); MURAKAMI, Akira, Moriguchi-shi Osaka 570-0083 (JP)
(74) Representative: Gardiner, Stephen Robin
(86) International application number: PCT/JP2007/074502
(87) International publication number: WO 2008/078643

(57) **Abstract**

A technique of adequately determining the position of the bone resection plane of artificial joint replacement and the size of the implant. The three-dimensional relative position relation (the three-dimensional position relation between the relation among frontal three elements and the relation among lateral three elements) among the five elements, i.e., a frontal The source of X-ray (FS), the plane (PS) to which the frontal radiograph belongs, a lateral The source of X-ray (FL), the plane (PL) to which the lateral radiograph belongs, a lateral The source of X-ray (FL), the plane (PL) to which the lateral radiograph belongs, and three-dimensional CAD data is determined (S7), shade images (IU, IL) of three dimensional CAD data on the implant are superimposed in an adequate position shown in the radiograph, and the images are displayed on a display (112) (S9, S10).

## Description

### TECHNICAL FIELD

The present invention relates to a surgical operation in which a joint of a patient with osteoarthritis or the like is replaced with an artificial joint, or artificial joint replacement. More particularly, the present invention relates to an assisting device for artificial joint replacement which is used to assist in such artificial joint replacement, and an assisting method for artificial joint replacement and an assisting system for artificial joint replacement which use the assisting device.

### BACKGROUND ART

Osteoarthritis is a disease which progresses with aging and in our country in which aging society is expected, an increase in the number of patients with osteoarthritis is presumed.
Artificial joint replacement is a surgical operation in which a joint of a patient with osteoarthritis or the like is bone resectiond, a bone surrounding the joint is resected and an implant is attached to the bone resection plane. It is often used particularly for aged patients as a very effective treatment technique.

In performing artificial joint replacement, prior to starting the surgical operation, the position of the implant and the bone resection plane are determined and a procedure called "templating (or "preoperative planning") to determine the adequate size of the implant to be attached to the bone resection plane is carried out as part of preoperative assistance.
In "templating," frontal and lateral radiographs of the patient' s joint to undergo artificial joint replacement are taken. Then, a template (hereinafter referred to as "two-dimensional template"), a transparent sheet on which the shape of an implant is drawn, is pressed on the joint position in the frontal radiograph and lateral radiograph to determine the adequate implant size and the bone resection plane.

In conventional templating which uses a two-dimensional template, the bone resection plane cannot be determined accurately unless the frontal radiograph and lateral radiograph are taken at the same magnification ratio.
Unless the plane of the frontal radiograph and the plane of the lateral radiograph are in a relative position relation so as to be perpendicular to each other, the position of the bone resection plane cannot be determined stereoscopically (three-dimensionally) accurately.

However, it is difficult to take radiographs so that the plane of a frontal radiograph and the plane of a lateral radiograph are in a relative position relation so as to be perpendicular to each other. For this reason, there exists a problem that the position, etc. of the bone resection plane determined by conventional templating is inaccurate.
Besides, in conventional templating which uses a two-dimensional template, the magnification ratio or reduction ratio cannot be corrected.
In consequence, there are cases that the position of the bone resection plane and the implant size as determined by conventional templating using a two-dimensional template must be corrected during a surgical operation for artificial joint replacement. In other words, there is a problem that conventional templating which uses a two-dimensional template is often not practically useful.

In conventional templating which uses a two-dimensional template, it may become necessary to take radiographs again. However, taking radiographs repeatedly involves the problem of radiation exposure of patients.
In addition, in case of a knee joint, if the patient has a flexion contracture in the knee, it is difficult even to take radiographs for conventional templating which uses a two-dimensional template. For a joint with a flexion contracture, the magnification ratio must be varied with regions of the joint, making it difficult to adjust the conventional two-dimensional template to the joint shown in the radiograph. In knee joints, a flexion contracture of 20-45 degrees is often seen.

As another conventional technique, a technique that the bone resection plane to which an implant is attached is calculated by a computer using X-ray films taken by X-ray equipment and data obtained by CT equipment has been proposed (see Patent Document 1).
Although this conventional technique is useful, it cannot be said to be satisfactory to assist in artificial joint replacement.

Also another technique has been proposed that in performing artificial joint replacement, a pin is driven into a lower thigh of a anesthetized patient, a base board is attached to the pin, metal plates are laminated on the base board and bone resection is performed on the uppermost surface of the laminate (see Patent Document 2).
Although this technique is also useful, it does not solve the above problem.
Patent Document 1: Japanese Unexamined Patent Publication No. 2004-8707
Patent Document 2: Japanese Unexamined Patent Publication No. 2004-8708

### DISCLOSURE OF THE INVENTION

### PROBLEM TO BE SOLVED BY THE INVENTION

The present invention has been proposed in view of the problems of the above related art and has an object to provide an assisting method for artificial joint replacement and an assisting system for artificial joint replacement in which the three-dimensional position of the implant, the bone resection plane position and the implant size can be determined adequately prior to artificial joint replacement, and an assisting device for artificial joint replacement for use therewith.

### MEANS FOR SOLVING THE PROBLEM

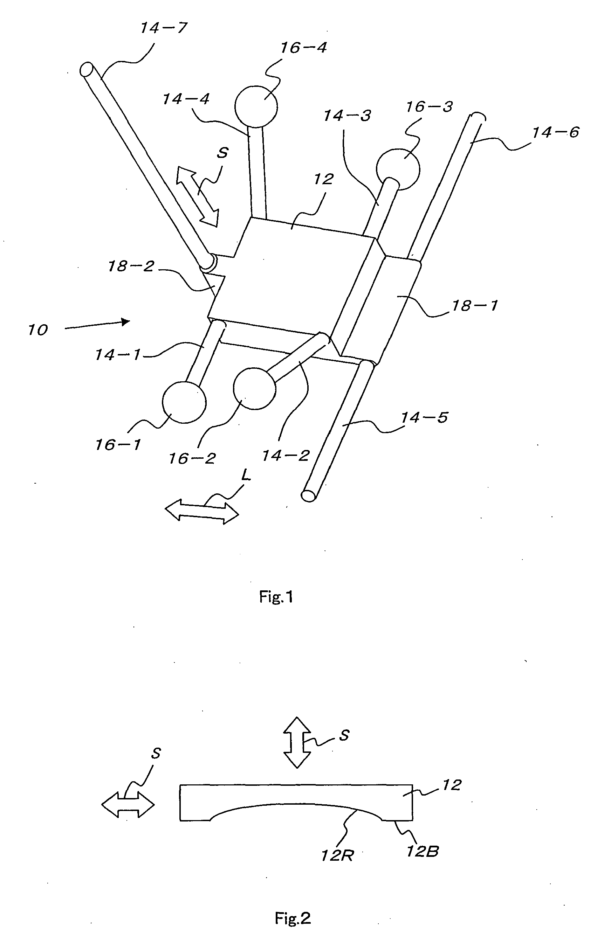

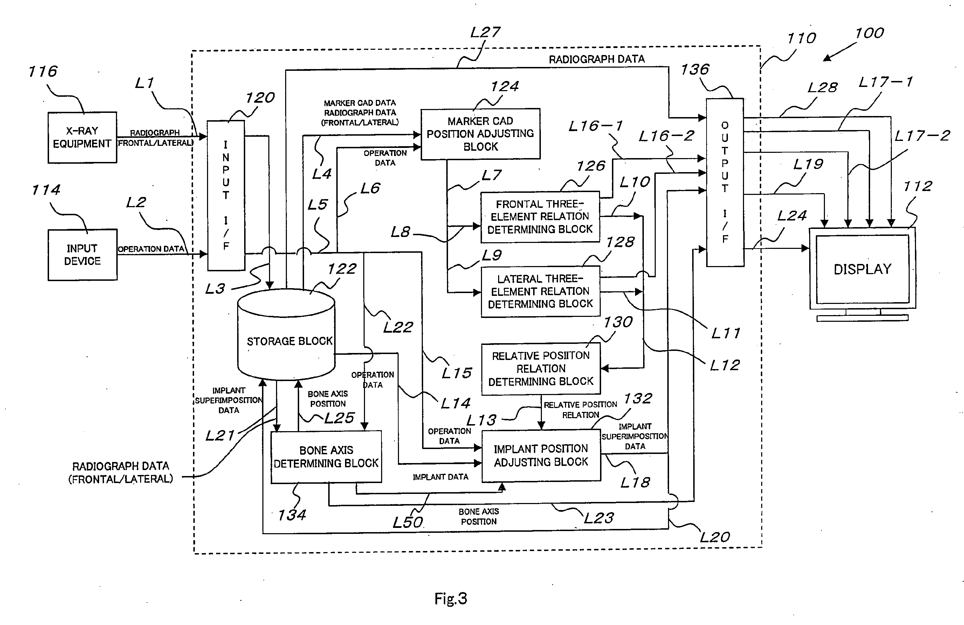

An assisting device for artificial joint replacement (marker 10) according to the present invention is characterized by including a main body (marker main body 12), four pins (14-1 to 14-4) attached to outer edges of the main body (12), horizontal pins (14-5, 14-6) attached to the main body through a first attachment flange (18-1) and extending horizontally (direction of arrow L), and a vertical pin (14-7) attached to the main body through a second attachment flange (18-2) and extending vertically (direction of arrow S), where the four pins (14-1 to 14-4) have ball-like pointing members (16-1 to 16-4) at their tips respectively, and the four pins (14-1 to 14-4) and the pointing members (16-1 to 16-4) are so arranged as not to overlap each other when seen from above and a vertical direction (direction of arrow S) (referred to as "frontal" in Fig. 3 onward)and a direction angled (with some inclination angle) to the vertical direction and not to overlap each other when seen from a horizontal direction (direction of arrow L: referred to as "lateral" in Fig. 3 onward) and a direction angled (with some inclination angle) to the horizontal direction (direction of arrow L) (Claim 1: Fig. 1).

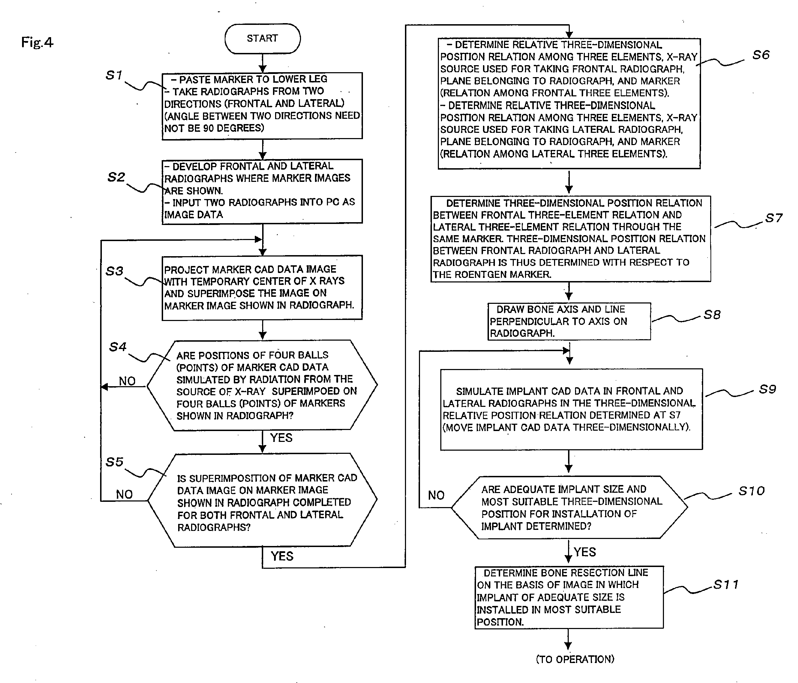

A computer-based assisting method for artificial joint replacement using the above assisting device for artificial joint replacement (marker 10) according to the present invention is characterized by including the steps of:
placing the assisting device for artificial joint replacement (10) in the vicinity of a patient' s joint to undergo artificial joint replacement and taking radiographs from two directions (for example, frontal and lateral) (Step S1);
putting three-dimensional CAD data (marker three-dimensional CAD data 10D) of the assisting device for artificial joint replacement (10) between an The source of X-ray and a radiograph (frontal radiograph and lateral radiograph), displaying it on a display (112), and superimposing a three-dimensional CAD data (10D) image (CAD image 10M) of the assisting device for artificial joint replacement (10) projected (10) in the radiograph (frontal radiograph and lateral radiograph), on an image (marker image 10S) of the assisting device for artificial joint replacement (10) projected in the radiograph (frontal radiograph and lateral radiograph) (Steps S3 to S5) ;
determining a relative three-dimensional position relation among an The source of X-ray for a frontal radiograph, three-dimensional CAD data (10D) of the assisting device for artificial joint replacement (10) and a frontal radiograph plane (relation among frontal three elements) and a relative three-dimensional position relation among an The source of X-ray for a lateral radiograph, three-dimensional CAD data (10D) of the assisting device for artificial joint replacement (10) and a lateral radiograph plane (relation among lateral three elements) (Step S6);
determining, from the relative three-dimensional position relation for the frontal radiograph ("relation among frontal three elements") and the relative three-dimensional position relation for the lateral radiograph ("relation among lateral three elements") as determined at the preceding step (Step S6), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) (Step S7); and
superimposing projected implant images (implant CAD images IU and IL) as there-dimensional CAD data in an adequate position of an image of the joint in a radiograph (frontal radiograph and lateral radiograph) in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined, and displaying it on the display (112) (Step S9, Step S10) (Claim 2: Fig. 4).

In this description, the term "computer" should be interpreted to imply not only personal computers and workstations but also a wide range of devices with a data processing function.

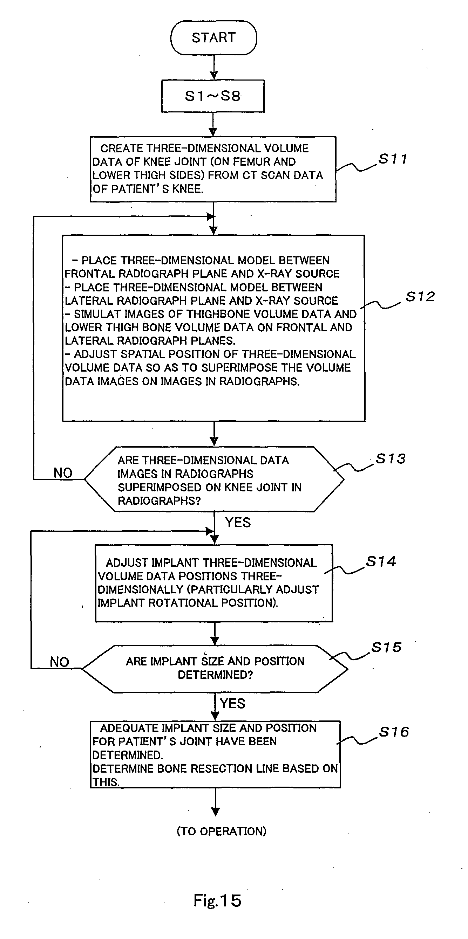

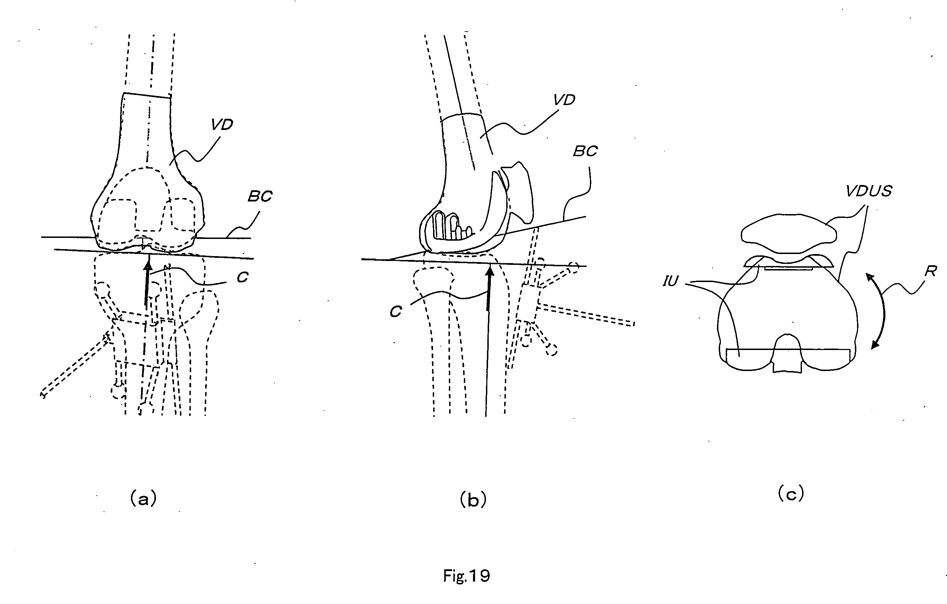

A computer-based assisting method for artificial joint replacement using the above assisting device for artificial joint replacement (marker 10) according to the present invention is characterized by including the steps of:
placing the assisting device for artificial joint replacement (10) in the vicinity of a patient's joint to undergo artificial joint replacement and taking radiographs from two directions (for example, frontal and lateral) (Step S1);
scanning the patient's joint to undergo artificial joint replacement by CT equipment (CT scanner 212) (Step S1);
putting three-dimensional CAD data (marker three-dimensional CAD data 10D) of the assisting device for artificial joint replacement (10) between an The source of X-ray and a radiograph (frontal radiograph and lateral radiograph), displaying it on a display (112), and superimposing a three-dimensional CAD data image (CAD image 10M) of the assisting device for artificial joint replacement (10) projected in the radiograph (frontal radiograph and lateral radiograph), on an image (marker image 10S) of the assisting device for artificial joint replacement (10) projected in the radiograph (frontal radiograph and lateral radiograph) (Steps S3 to S5);
determining a relative three-dimensional position relation among an The source of X-ray for a frontal radiograph, three-dimensional CAD data (10D) of the assisting device for artificial joint replacement (10) and a frontal radiograph plane (relation among frontal three elements) and a relative three-dimensional position relation among an The source of X-ray for a lateral radiograph, three-dimensional CAD data (10D) of the assisting device for artificial joint replacement (10) and a lateral radiograph plane (relation among lateral three elements) (Step S6);
determining, from the relative three-dimensional position relation for the frontal radiograph ("relation among frontal three elements") and the relative three-dimensional position relation for the lateral radiograph ("relation among lateral three elements") as determined at the preceding step (Step S6), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) (Step S7);
creating, from data obtained by scanning the patient's joint to undergo artificial joint replacement by CT equipment (CT scanner 212), three-dimensional volume data (VDU, VDL) including three-dimensional data of the patient's joint (Step S11);
putting the three-dimensional volume data (VDU, VDL) between an The source of X-ray and a radiograph (frontal radiograph and lateral radiograph) and superimposing three-dimensional volume data images (volume data images VDUS, VDLS) projected in the radiograph (frontal radiograph and lateral radiograph), on a joint part shown in the radiograph (frontal radiograph and lateral radiograph) (Steps S12 and S13); and
adjusting rotational position (position in the direction of arrow R in Fig. 19(c)) of the implant as three-dimensional CAD data to superimpose implant images as the there-dimensional CAD data (implant CAD images IU and IL) in an adequate position of the joint in a radiograph (frontal radiograph and lateral radiograph) in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined and the three-dimensional volume data images (VDUS, VDLS) are superimposed on the joint part, and displaying it on the display (112) (Steps S14 and S15) (Claim 3: Fig. 15).

An assisting system for artificial joint replacement using the assisting device for artificial joint replacement (marker 10) according to the present invention is characterized by including:
a system main unit (110) which receives radiographs (frontal radiograph and lateral radiograph) taken from two directions (for example, frontal and lateral) with the assisting device for artificial joint replacement (10) placed in the vicinity of a patient's joint to undergo artificial joint replacement, and a display (112),
where the system main unit (110) includes:
a device designed to put three-dimensional CAD data (marker three-dimensional CAD data 10D) of the assisting device for artificial joint replacement (10) between an The source of X-ray and a radiograph (frontal radiograph and lateral radiograph) and superimpose a three-dimensional CAD data image (CAD image 10M) of the assisting device for artificial joint replacement (10) projected in the radiograph (frontal radiograph and lateral radiograph), on an image (marker image 10S) of the assisting device for artificial joint replacement (10) projected in the radiograph (frontal radiograph and lateral radiograph) (marker CAD position adjusting block 124);
a device designed to determine a relative three-dimensional position relation among an The source of X-ray for a frontal radiograph, three-dimensional CAD data (10D) of the assisting device for artificial joint replacement (10) and a frontal radiograph plane (relation among frontal three elements) (frontal three-element relation determining block 126);
a device designed to determine a relative three-dimensional position relation among an The source of X-ray for a lateral radiograph, three-dimensional CAD data (10D) of the assisting device for artificial joint replacement (10) and a lateral radiograph plane (relation among lateral three elements) (lateral three-element relation determining block 128);
a device designed to determine, from the relative three-dimensional position relation for the frontal radiograph (relation among frontal three elements) and the relative three-dimensional position relation for the lateral radiograph (relation among lateral three elements), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) (relative position relation determining block 130); and
a device designed to superimpose implant images as there-dimensional CAD data (implant CAD images IU and IL)in an adequate position of the j oint in a radiograph (frontal radiograph and lateral radiograph) in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three element relation and the lateral three-element relation) has been determined, and display it on the display (112) (implant position adjusting block 132) (Claim 4: Fig. 3).

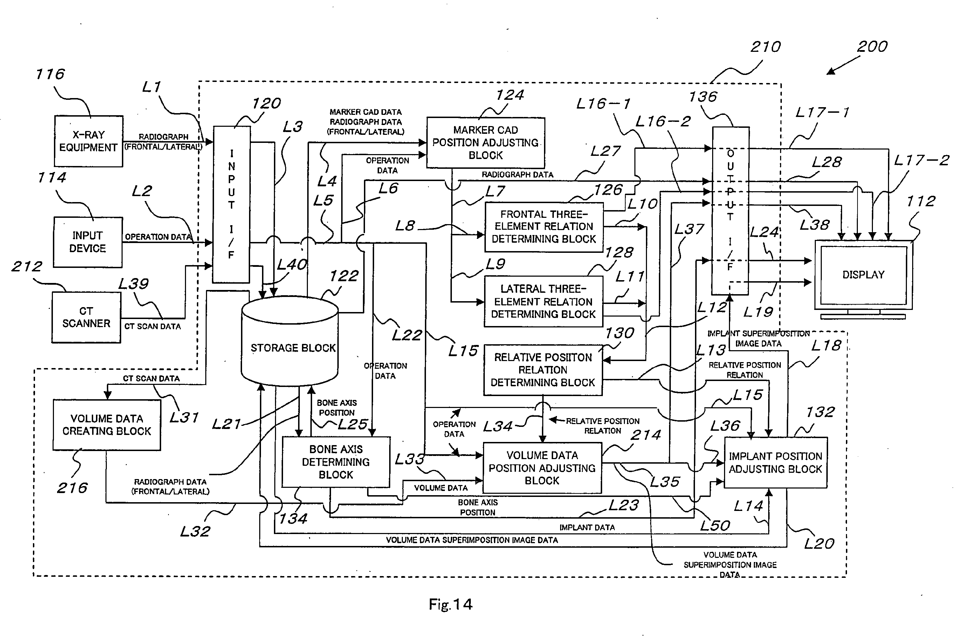

Alternatively an assisting system for artificial joint replacement using the assisting device for artificial joint replacement (marker 10) according to the present invention is
characterized by including a system main unit (110) and a display (112), where
the system main unit (110) is designed to receive radiographs (frontal radiograph and lateral radiograph) taken from two directions (for example, frontal and lateral) with the assisting device for artificial joint replacement (10) placed in the vicinity of a patient's joint to undergo artificial joint replacement and receive scan data obtained by scanning the patient's joint to undergo artificial joint replacement by CT equipment (CT scanner 212); and
the system main unit (110) includes:
a device designed to put three-dimensional CAD data (marker three-dimensional CAD data 10D) of the assisting device for artificial joint replacement (10) between an The source of X-ray and a radiograph (frontal radiograph and lateral radiograph) and superimpose a three-dimensional CAD data image (CAD image 10M) of the assisting device for artificial joint replacement (10) projected in the radiograph (frontal radiograph and lateral radiograph), on an image (marker image 10S) of the assisting device for artificial joint replacement (10) projected in the radiograph (frontal radiograph and lateral radiograph) (marker CAD position adjusting block 124);
a device designed to determine a relative three-dimensional position relation among an The source of X-ray for a frontal radiograph, three-dimensional CAD data (10D) of the assisting device for artificial joint replacement (10) and a frontal radiograph plane (relation among frontal three elements) (frontal three-element relation determining block 126);
a device designed to determine a relative three-dimensional position relation among an The source of X-ray for a lateral radiograph, three-dimensional CAD data (10D) of the assisting device for artificial joint replacement (10) and a lateral radiograph plane (relation among lateral three elements) (lateral three-element relation determining block 128);
a device designed to determine, from the relative three-dimensional position relation for the frontal radiograph (relation among frontal three elements) and the relative three-dimensional position relation for the lateral radiograph (relation among lateral three elements), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) (relative position relation determining block 130);
a device designed to create, from data obtained by scanning the patient's joint to undergo artificial joint replacement by CT equipment (CT scanner 212), three-dimensional volume data (VDU, VDL) including three-dimensional data of the patient's joint (volume data creating block 216);
a device designed to put the three-dimensional volume data (VDU, VDL) between an The source of X-ray and a radiograph (frontal radiograph and lateral radiograph), display it on the display (112) and superimpose three-dimensional volume data images (volume data images VDUS, VDLS) projected on the plane of the radiograph (frontal radiograph and lateral radiograph), on a joint part shown in the radiograph (frontal radiograph and lateral radiograph) (volume data position adjusting block 214); and
a device designed to adjust rotational position (position in the direction of arrow R in Fig. 19(c)) of the implant as three-dimensional CAD data to superimpose the implant images as there-dimensional CAD data (implant CAD images IU and IL) in an adequate position of the joint in a radiograph (frontal radiograph and lateral radiograph) in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined and the three-dimensional volume data images (VDUS, VDLS) are superimposed on the joint part, and display it on the display (112) (implant position adjusting block 132) (Claim 5: Fig. 14).

A computer program which enables a computer to carry out the artificial joint replacement method (Claim 2) using the assisting device for artificial joint replacement (marker 10) according to the present invention is designed to perform the steps of:
inputting radiographs (frontal radiograph and lateral radiograph) taken from two directions (for example, frontal and lateral) with the assisting device for artificial joint replacement (10) placed in the vicinity of a patient's joint to undergo artificial joint replacement (Step S2);
putting three-dimensional CAD data (marker three-dimensional CAD data 10D) of the assisting device for artificial joint replacement (10) between an The source of X-ray and a radiograph (frontal radiograph and lateral radiograph), and displaying it on a display (112) (Step S3);
superimposing a three-dimensional CAD data image (CAD image 10M) of the assisting device for artificial joint replacement (10) projected (10) in the radiograph (frontal radiograph and lateral radiograph), on an image (marker image 10S) of the assisting device for artificial joint replacement projected in the radiograph (frontal radiograph and lateral radiograph) in response to operation data entered through an input device (114) (Steps S3 to S5);
determining a relative three-dimensional position relation among an The source of X-ray for a frontal radiograph, three-dimensional CAD data (10D) of the assisting device for artificial joint replacement (10) and a frontal radiograph plane (relation among frontal three elements) and a relative three-dimensional position relation among an The source of X-ray for a lateral radiograph, three-dimensional CAD data (10D) of the assisting device for artificial joint replacement (10) and a lateral radiograph plane (relation among lateral three elements) (Step S6);
determining, from the relative three-dimensional position relation for the frontal radiograph ("relation among frontal three elements") and the relative three-dimensional position relation for the lateral radiograph ("relation among lateral three elements") as determined at the preceding step (Step S6), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) (Step S7); and
superimposing projected implant images as there-dimensional CAD data (implant CAD images IU and IL) in an adequate position of an image of the joint in a radiograph (frontal radiograph and lateral radiograph) in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined, and displaying it on the display (112)(Step S9, Step S10) (Claim 6: Fig. 4).

Also, a computer program which enables a computer to carry out the artificial joint replacement method (Claim 3) using the assisting device for artificial joint replacement (marker 10) according to the present invention is designed to perform the steps of:
inputting radiophotographs taken from two directions with the assisting device for artificial joint replacement (10) as described in Claim 1 placed in the vicinity of a patient's joint to undergo artificial joint replacement, and inputting data obtained by scanning the patient's joint to undergo artificial joint replacement by CT equipment (CT scanner 212) (Step S2);
putting three-dimensional CAD data (marker three-dimensional CAD data 10D) of the assisting device for artificial joint replacement (10) between an The source of X-ray and a radiograph (frontal radiograph and lateral radiograph), displaying it on a display (112), and superimposing a three-dimensional CAD data (10D) image (CAD image 10M) of the assisting device for artificial joint replacement (10) projected in the radiograph (frontal radiograph and lateral radiograph), on an image (marker image 10S) of the assisting device for artificial joint replacement (10) projected in the radiograph (frontal radiograph and lateral radiograph) (Steps S3 to S5);
determining a relative three-dimensional position relation among an The source of X-ray for a frontal radiograph, three-dimensional CAD data (10D) of the assisting device for artificial joint replacement (10) and a frontal radiograph plane (relation among frontal three elements) and a relative three-dimensional position relation among an The source of X-ray for a lateral radiograph, three-dimensional CAD data (10D) of the assisting device for artificial joint replacement (10) and a lateral radiograph plane (relation among lateral three elements) (Step S6);
determining, from the relative three-dimensional position relation for the frontal radiograph ("relation among frontal three elements") and the relative three-dimensional position relation for the lateral radiograph ("relation among lateral three elements") as determined at the preceding step (Step S6), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) (Step S7);
creating, from data obtained by scanning the patient's joint to undergo artificial joint replacement by CT equipment (CT scanner 212), three-dimensional volume data (VDU, VDL) including three-dimensional data of the patient's joint (Step S11);
putting the three-dimensional volume data (VDU, VDL) between an The source of X-ray and a radiograph (frontal radiograph and lateral radiograph) plane and superimposing three-dimensional volume data images (volume data images VDUS, VDLS) projected in the radiograph (frontal radiograph and lateral radiograph) on a joint part shown in the radiograph (frontal radiograph and lateral radiograph) (Steps S12 and S13); and
adjusting rotational position (position in the direction of arrow R in Fig. 19(c)) of the implant as three-dimensional CAD data to superimpose the implant images as there-dimensional CAD data (implant CAD images IU and IL) in an adequate position of the joint in a radiograph (frontal radiograph and lateral radiograph) in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined and the three-dimensional volume data images (VDUS, VDLS) are superimposed on the joint part, and displaying it on the display (112) (Steps S14 and S15) (Claim 7: Fig. 15).

### EFFECT OF THE INVENTION

According to the assisting method for artificial joint replacement and assisting system for artificial joint replacement or computer program according to the present invention as designed as mentioned above, after a relative three-dimensional position relation among an The source of X-ray, three-dimensional CAD data (10D) of the assisting device for artificial joint replacement (10) and a frontal radiograph (relation among frontal three elements) and a relative three-dimensional position relation among an The source of X-ray, three-dimensional CAD data (10D) of the assisting device for artificial joint replacement (10) and a lateral radiograph (relation among lateral three elements) are determined, a three-dimensional relative position relation among five elements, i.e., the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) is determined.
Therefore, according to the present invention, the three-dimensional position relation can be determined accurately even if the frontal radiograph is not exactly perpendicular to the lateral radiograph. Specifically, unlike the conventional case that a two-dimensional template is used, the three-dimensional position relation can be determined accurately even if the angle between the frontal radiograph and the lateral radiograph is not exactly 90 degrees (not perpendicular).

Furthermore, according to the present invention, when the three-dimensional position relations for the frontal radiograph and lateral radiograph have been determined, the adequate implant position can be determined. Specifically, if the implant images (IU, IL) as three-dimensional CAD data in one radiograph (for example, frontal radiograph) are moved, the implant images as three-dimensional CAD data in the other (for example, lateral radiograph) are also moved in conjunction therewith.
Besides, if a different size implant image (IU) as three-dimensional CAD data is projected in one radiograph (for example, frontal radiograph), an implant image of the different size as three-dimensional CAD data is also projected in the other radiograph (for example, lateral radiograph).

In the related art in which only two-dimensional templating can be performed, templating for the frontal radiograph and templating for the lateral radiograph must be performed separately in order to determine the adequate implant position and there are cases that the implant position in the frontal radiograph does not match that in the lateral radiograph three-dimensionally.
On the other hand, according to the present invention, when a three-dimensional relative position relation among five elements, i.e., the frontal The source of X-ray (FS), plane (PS) belonging to the frontal radiograph, lateral The source of X-ray (FL), plane (PL) belonging to the lateral radiograph, and three-dimensional CAD data (10D), (three-dimensional position relation between the frontal three-element relation and the lateral three-element relation) has been determined, the implant size and position with respect to the joint in the radiograph can be adjusted, namely three-dimensional templating can be performed.

Furthermore, according to the present invention, since templating is performed while a three-dimensional relative position relation among five elements, i.e., the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined, the doctor or operator can determine the bone resection line easily and accurately.
In other words, according to the present invention, a simulation of a postoperative radiograph of artificial joint replacement as a target which the doctor or operator should achieve is displayed.

In the present invention, when, from data obtained by scanning a patient's joint to undergo artificial joint replacement by CT equipment (CT scanner 212), three-dimensional volume data (VDU, VDL) including three-dimensional data of the patient's joint is created, and three-dimensional volume data images (VDUS, VDLS) projected in the radiograph (frontal radiograph and lateral radiograph) are superimposed on a joint part shown in the radiograph (frontal radiograph and lateral radiograph), and the rotational position (position in the direction of arrow R in Fig. 19(c)) of the implant as three-dimensional CAD data is adjusted to superimpose implant images (IU, IL) as the there-dimensional CAD data in an adequate position of the joint in the radiograph (frontal radiograph and lateral radiograph) (Claims 3, 5 and 7), the implant position as three-dimensional CAD data regarding so-called rotation (in the direction of arrow R in Fig. 19 (c)) can be adjusted, so the implant position can be determined with further accuracy.

In the present invention, in order to determine the three-dimensional position relation between the frontal radiograph and lateral radiograph with respect to the roentgen marker, it is necessary to superimpose the three-dimensional CAD data image (10M) of the artificial joint replacement device (10) projected in the radiograph (frontal radiograph and lateral radiograph), on the image of the assisting device for artificial joint replacement (10) projected in the radiograph (frontal radiograph and lateral radiograph).
In this process, a decision can be made as to whether or not the parts corresponding to the ball-like pointing members 16-1 to 16-4 at the tips of the pins 14-1 to 14-4 in the image (10S) of the assisting device for artificial joint replacement (10) projected in the radiograph (frontal radiograph and lateral radiograph) are overlapping those in the three-dimensional CAD data image (10M) of the assisting device for artificial joint replacement (10) projected in the radiograph (frontal radiograph and lateral radiograph).
In other words, by using the assisting device for artificial joint replacement (marker 10) according to the present invention, the process required to determine the three-dimensional position relation between the frontal radiograph and lateral radiograph with respect to the roentgen marker can be carried out easily and accurately.
The term "artificial joint" used in this description implies all kinds of artificial joints including artificial knee joints, artificial hip joints and artificial elbow joints.

### BEST MODE FOR CARRYING OUT THE INVENTION

Next, embodiments of the present invention will be described referring to the accompanying drawings.
First, an assisting device for artificial joint replacement (roentgen marker or maker: 10) according to an embodiment of the present invention is described below referring to Fig. 1.

In Fig. 1, the marker, the whole of which is designated by reference numeral 10, is comprised of four pins 14-1 to 14-4, horizontal pins 14-5 and 14-6, and a vertical pin 14-7 which are fitted to a marker body 12 almost in the form of a rectangular parallelepiped. Here, the horizontal pin 14-5 and horizontal pin 14-6 may be two separate round bar members or a single round bar member.
As shown in Fig. 2, a curved concave 12R is formed on the bottom surface 12B of the marker body 12, namely the surface to contact the patient body (not shown in Fig. 1), in a way to fit the patient body. When the marker 10 is attached to the patient body, the curved concave 12R closely contacts the patient body and prevents the marker 10 from becoming "unstable."
Here, for simpler illustration, the pins 14-1 to 14-7, pointing members 16-1 to 16-4 and so on are omitted in Fig. 2.
Though not shown, the shape of the marker body 12 is not limited to the rectangular parallelepiped.

The pins 14-1 to 14-4 have ball-like pointing members 16-1 to 16-4 at their tips. Though not shown clearly in Fig. 1, the ball-like pointing members 16-1 to 16-4 may be different in color.
The horizontal pins 14-5 and 14-6 are attached to the marker body 12 through a first attachment flange 18-1. The vertical pin 14-7 is attached to the marker body 12 through a second attachment flange 18-2.

In Fig. 1, the pin 14-5 and pin 14-6 extend parallel to the plane where the marker 10 is placed (in the horizontal direction) and in Fig. 1, the horizontal direction is indicated by arrow L.
In Fig. 1, the pin 14-7 extends vertically to the plane
where the marker 10 is placed (in the vertical direction) . In Figs. 1 and 2, the direction in which the pin 14-7 extends (vertical direction) is indicated by arrow S.

In the marker shown in Fig. 1, the four pins 14-1 to 14-4 have the same length but extend in different directions. Here, it is preferable that the four pins 14-1 to 14-4 be different in length. This is because if the four pins 14-1 to 14-4 are different in length, the pins 14-1 to 14-4 hardly overlap each other when the marker 10 is observed in a mode which will be stated later.
Though not clear in Fig. 1, when seen from above (frontal direction) and the vertical direction (direction of arrow S), the pins 14-1 to 14-4 are arranged so that the ball-like pointing members 16-1 to 16-4 at their tips do not overlap each other. In addition, when observed from above and a direction somewhat angled to the vertical direction (direction of arrow S), they are also arranged so that the ball-like pointing members 16-1 to 16-4 do not overlap each other:

Also, when seen from the horizontal direction (direction of arrow L: lateral direction), the pins 14-1 to 14-4 are arranged so that the ball-like pointing members 16-1 to 16-4 at their tips do not overlap each other. In addition, when observed from a direction somewhat angled to the horizontal direction (direction of arrow L), they are also arranged so that the ball-like pointing members 16-1 to 16-4 do not overlap each other.

Here, regarding the vertical direction (direction of arrow S) and the horizontal direction (direction of arrow L), the expression "direction somewhat angled" must be considered case by case. However, in three-dimensional templating using the marker 10 shown in Fig. 1 which is described below referring to Fig. 3 onward (implementation of the assisting method for artificial joint replacement and assisting system for artificial joint replacement), the direction as a frontal direction or lateral direction has no problem if it is within the bounds of common sense of a surgeon who performs artificial joint replacement.
In other words, the pins 14-1 to 14-4 are arranged so that the ball-like pointing members 16-1 to 16-4 do not overlap each other when seen from two directions almost perpendicular to each other (almost vertical direction and almost horizontal direction).

The pins 14-5 to 14-7, which have no ball-like pointing members at their tips, are used to find the three-dimensional relative position relation (solution) among three elements, i.e., an The source of X-ray used for taking a radiograph, the plane of the radiograph and three-dimensional CAD data, as will be stated later. In other words, two types of "solution" can be found by a process which will be described later and the doctor or operator selects the solution that the positions of the pins 14-5 to 14-7, which have no ball-like pointing members at their tips, coincide.
Also, deformation of the ball-like pointing members 16-1 to 16-4 or overall deformation of the marker 10 due to aging can be detected by checking the pins 14-5 to 14-7 which have no ball-like pointing members at their tips.

In assisting in performing artificial joint replacement, the marker 10 is used in templating, a kind of preoperative assistance.
In performing templating, the marker 10 is attached to the vicinity of a joint of a patient (for example, the front of the tibia when it is used to assist in artificial knee joint replacement). More specifically, for example, if it is a knee joint, the marker 10 is attached in a way that the bottom surface of the marker 10 (surface not shown in Fig. 1) contacts a region below the junction of the tibia and fibula on the front of the patient's joint.
What kind of role the marker 10 plays in templating will be detailed referring to Fig. 3 onward in the explanation of an assisting method for artificial joint replacement and an assisting system for artificial joint replacement according to embodiments of the present invention.

Next, the assisting method for artificial joint replacement and assisting system for artificial joint replacement according to an embodiment of the present invention will be described referring to Fig. 3 onward.
First, referring to Figs. 3 to 13, the assisting method for artificial joint replacement and assisting system for artificial joint replacement according to a first embodiment are described below.
Fig. 3 is a bloc diagram of the assisting system for artificial joint replacement.
In Fig. 3, the assisting system for artificial joint replacement, the whole of which is designated by reference numeral 100, is mainly comprised of: a system main unit 110 composed of a computer; a display 112 as a display unit; input means 114 composed of a keyboard and a mouse or the like; and X-ray equipment 116 as a system for taking radiographs.

The system main unit 110 includes an input interface 120, a storage block 122 as a database, a marker CAD position adjusting block 124, a frontal three-element relation determining block 126 and a lateral three-element relation determining block 128, a relative position relation determining block 130, an implant position adjusting block 132, a bone axis determining block 134, and an output interface 136.
These blocks 120 to 136 in the system main unit 110 are arranged so that signals or information can be transmitted through lines.

The storage block 122 as a database stores image data of radiographs taken by the X-ray equipment 116 through line L1, the input interface 120 and line L3.
Here, line L1 not only represents a communication cable but also implies wireless communications. It also implies that data is stored in a storage medium such as CD-ROM and the storage medium is carried by the doctor or operator.
As shown in Fig. 3, after the image data of a radiograph taken by the X-ray equipment 116 (image data of a radiograph taken with the marker 10 attached to the joint) is stored in the storage block 122, it is sent to the marker CAD position adjusting block 124 through line L4. Instead, the image data of the radiograph may be passed from the X-ray equipment 116 through line L1 and the input interface 120 without going through the storage block 122 and sent directly to the marker CAD position adjusting block 124 through a line (not shown).

The frontal radiograph data and lateral radiograph data stored in the storage block 122 can be displayed on the display 112 through line L27, the output interface 136, and line L28.
Here, the enlargement ratio, reduction ratio, brightness and.density can be freely specified by electronically inputting the frontal radiograph data and lateral radiograph data into the system main unit 110 composed of a computer or the like.

The storage block 122 also stores three-dimensional CAD data of the roentgen marker 10 described in reference to Fig. 1. For this three-dimensional CAD data, the ball-like pointing members 16-1 to 16-4 at the tips of the pins 14-1 to 14-4 are colored. The storage block 122 also stores three-dimensional CAD data of the implant.
As will be described later, position information on the bone axis (functional axis, anatomical axis) determined by the bone axis determining block 134 is also stored in the storage block 122.

Three-dimensional CAD data of the roentgen marker 10 as well as radiograph image data is sent from the storage block 122 to the marker CAD position adjusting block 124 through line L4. At the same time, operation information entered by the doctor or operator in charge of the operation through the input device 114 (operation data) is sent to the marker CAD position adjusting block 124 through line L2, the input interface 120, line L5 and line L6.

As will be described later referring to Fig. 4 and subsequent drawings, in the marker CAD position adjusting block 124, an image of the marker 10 projected on the plane of a radiograph (hereinafter "marker image") and an image of the marker 10, as three-dimensional CAD data, projected on a radiograph by a temporary The source of X-ray (center of X ray radiation) (hereinafter "CAD image") are superimposed one upon the other to determine the relative three-dimensional position relation among three elements, i.e., the The source of X-ray used for taking a radiograph, radiograph plane and marker three-dimensional CAD data ("relation among three elements"). Such relative three-dimensional position relation ("relation among three elements") is sent to the relative position relation determining block 130.

The marker CAD position adjusting block 124 is connected to the frontal three-element relation determining block 126 and the lateral three-element relation determining block 128 through lines L7, L8 and L9.
For a radiograph of the front (direction of arrow S in Fig. 1) of the joint of the patient to which the marker 10 is attached, the frontal three-element relation determining block 126 determines the relative three-dimensional position relation among three elements, i.e., the The source of X-ray used for taking the radiograph, the plane of the radiograph and three-dimensional CAD data of the marker 10 ("relation among frontal three elements").
Then, for a radiograph of the lateral side (direction of arrow L in Fig. 1) of the joint of the patient to which the marker 10 is attached, the lateral three-element relation determining block 128 determines the relative three-dimensional position relation among three elements, i.e., the The source of X-ray used for taking the radiograph, the plane of the radiograph and three-dimensional CAD data of the marker 10 ("relation among lateral three elements").

Regarding the frontal three-element relation determining block 126, movement of the CAD image (10M) to superimpose the CAD image on the marker image on the frontal radiograph plane is displayed on the display 112 through line L16-1, the output interface 136, and line L17-1.
On the other hand, regarding the lateral three-element relation determining block 128, movement of the CAD image (10M) to superimpose the CAD image on the marker image and CAD image on the lateral radiograph plane is displayed on the display 112 through line L16-2, the output interface 136, and line L17-2.

Here, the three-dimensional CAD data of the marker 10 in the "relation among frontal three elements" is the same as the three-dimensional CAD data of the marker 10 in the "relation among lateral three elements." Therefore, the "relation among frontal three elements" and the "relation among lateral three elements" can be correlated three-dimensionally using the known software technique called "The software 'PhotoModeler'."

Once the "relation among frontal three elements" and "relation among lateral three elements" have been determined in the frontal three-element relation determining block 126 and lateral three-element relation determining block 128, the "relation among frontal three elements" and "relation among lateral three elements" are sent to the relative position relation determining block 130 through lines L10, L11 and L12.
The relative position relation determining block 130 is designed to find the three-dimensional position relation between the "relation among frontal three elements" and "relation among lateral three elements" (frontal three-element relation and lateral three-element relation) in the abovementioned mode. After the three-dimensional position relation between the "relation among frontal three elements" and "relation among lateral three elements" has been determined, as the CAD image in either the frontal radiograph or the lateral radiograph is moved, the CAD image in the other is moved in conjunction therewith.

Once the three-dimensional position relation between the "relation among frontal three elements" and "relation among lateral three elements" (frontal three-element relation and lateral three-element relation) has been determined in the relative position relation determining block 130, such three-dimensional position relation is sent to the implant position adjusting block 132 through line L13.
In artificial joint replacement, the implant position adjusting block 132 determines the position of the artificial joint (implant) to replace the patient's joint. Specifically, the size of the implant attached to the patient's joint and its three-dimensional position with respect to the patient's bone are determined.

The three-dimensional position relation between the frontal thee-element relation and lateral thee-element relation is sent to the implant position adjusting block 132 and three-dimensional CAD data of the implant is also sent from the storage block 122 to it through line L14.
In addition, operation information entered by the doctor or operator in charge of operation through the input device 114 (operation data) is sent to the implant position adjusting block 132 through line L2, the input interface 120, line L5 and line L15.
As will be described later referring to Fig. 4 and subsequent drawings, the doctor or operator adjusts the three-dimensional CAD data of the implant (more particularly an image of the three-dimensional CAD data of the implant as projected on the same plane as the radiograph by X rays from the The source of X-ray : implant CAD image) so as to place it in an adequate position of the patient's joint shown in the radiograph. Operation data concerning such adjustment is sent to the implant position adjusting block 132 as data for moving the implant CAD image.

Movement of the tree-dimensional CAD data of the implant (its image in the radiograph) to place it in an adequate position of the patient's joint and how it is placed in the adequate position of the patient's joint in the radiograph are displayed on the display 112 through line L18, the output interface 136 and line L19.
At the same time, data on how the three-dimensional CAD data of the implant (its image in the radiograph) is placed in the adequate position of the patient' s joint is sent to the storage block 122 through line L120 and stored in it.

In Fig. 3, reference numeral 134 represents a bone axis determining block. The bone axis determining block 134 has the function of showing a bone axis such as the functional axis or anatomical axis of a femur or tibia in a frontal radiograph or lateral radiograph.
For example, indication of the bone axis in a radiograph (data indicating it) is very helpful in placing the three-dimensional CAD data of the implant (its image in the radiograph) in an adequate position of the joint or determining the bone resection plane. For this reason, the embodiment shown here is designed to indicate the bone axis in a frontal radiograph and a lateral radiograph.
Here, the bone axis data determined in the bone axis determining block 134 is sent to the implant position adjusting block 132 through line L50. The implant position adjusting block 132 has the function of adjusting the three-dimensional CAD data of the implant (its image in the radiograph) to a position perpendicular to the bone axis selectively in response to the operator's request.

Frontal and lateral photograph data are sent from the storage block 122 to the bone axis determining block 134 through line 21.
At the same time, operation data concerning bone axis determination entered by the doctor or operator through the input device 114 is sent to the bone axis determining block 134 through line L2, the input interface 120, line L5 and line 22.

The bone axis determined in the bone axis determining block 134 is sent to the display 112 through line L23, the output interface 136 and line L24 while being shown in the frontal and lateral radiographs so that the doctor or operator can check it. At the same time, the data on the bone axis shown in the frontal and lateral radiographs is sent to the storage block 122 through line L25 and stored in it.

The sequence of assisting in artificial joint replacement using the assisting system for artificial joint replacement 100 shown in Fig. 3, specifically the sequence of performing templating, is explained below referring to Figs. 4 to 13.

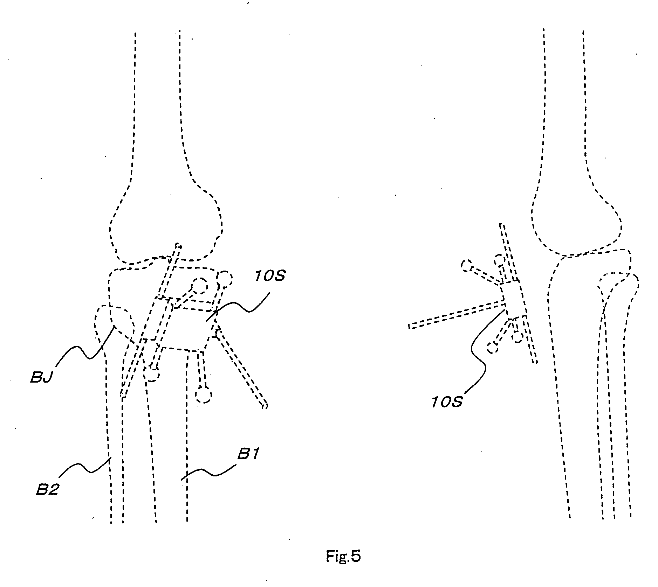

In Fig. 4, at Step S1, the marker 10 is fixed in the vicinity of the joint BJ on the front of the patient's joint (stated later) as the object of artificial joint replacement, namely as shown on the left (frontal radiograph) in Fig. 5 and a frontal radiograph (left in Fig. 5) and a lateral radiograph (right in Fig. 5) are taken.
Here it is unnecessary that the plane of the frontal radiograph and the plane of the lateral radiograph be exactly perpendicular to each other. In other words, while templating in the related art requires the frontal radiograph and lateral radiograph to be exactly perpendicular to each other, in this sequence the frontal and lateral radiographs need not be perpendicular to each other because three-dimensional position relations can be calculated using the marker 10.

Next, the frontal radiograph (left in Fig. 5) and lateral radiograph (right in Fig. 5) thus taken are inputted into the system main unit 110 shown in Fig. 3 as electronic data (Step S2). Such electronic data is stored in the storage block 122 (Fig. 3).
An example of radiographs inputted as electronic data is shown in Fig. 5. In Fig. 5, the image of the marker 10 projected on the radiograph plane (marker image) is designated by reference numeral 10S.
In Fig. 4, the steps after Step S2 are all carried out in the system main unit 110 (for example, computer) and how they are being carried out are displayed on the display 112 (Fig. 3).

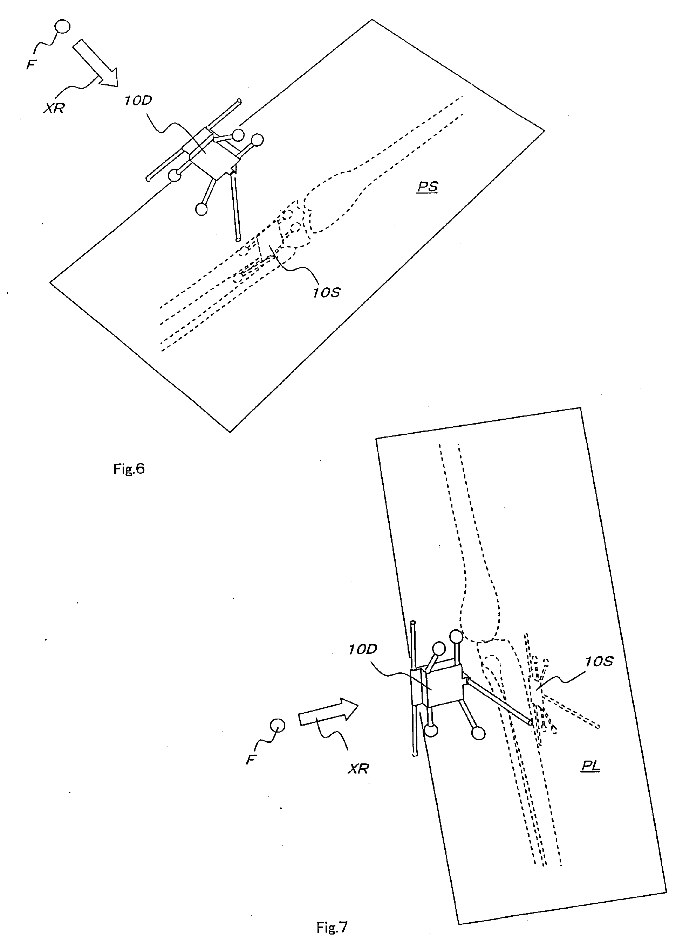

At Step S3, operation is done using the marker three-dimensional CAD data stored in the storage block 122 in the marker CAD position adjusting block 124 (Fig. 3).
Specifically, as shown in Figs. 6 and 7, images of the marker as three-dimensional CAD data 10D (images projected on the same planes PS, PL as the radiograph data: CAD images positionally unadjusted) projected on the same planes (planes PS, PL in Figs. 6 and 7) as the radiographs (data) are simulated where irradiation is made with X rays XR from a temporary center of X rays F (temporarily determined center of X rays before the original center of X rays is determined).

In this situation, the doctor or operator moves and adjusts the three-dimensional position of the marker as three-dimensional CAD data 10D so that the image of the marker as three-dimensional CAD data 10D projected on the radiograph plane (CAD image 10M) is superimposed on the marker image 10S projected in the radiograph.
In Figs. 6 to 9, the image of the marker as three-dimensional CAD data 10D projected on the plane PS, PL (CAD image) is designated by reference numeral 10M.

How an image of the marker as three-dimensional CAD data 10D is projected on the plane PS, PL is explained below referring to Figs. 6 to 9.
Fig. 6 shows that the marker as three-dimensional CAD data 10D is irradiated with X rays XR from the temporary center of X rays where the X rays are directed toward the same plane PS as the frontal radiograph data. On the other hand, Fig. 7 shows that the marker as three-dimensional CAD data 10D is irradiated with X rays XR from the temporary center of X rays where the X rays are directed toward the same plane PL as the lateral radiograph data.

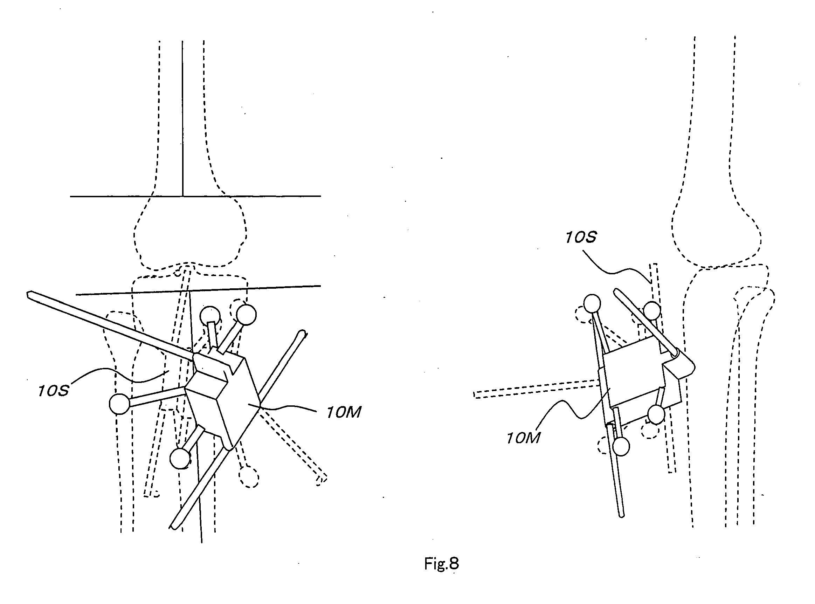

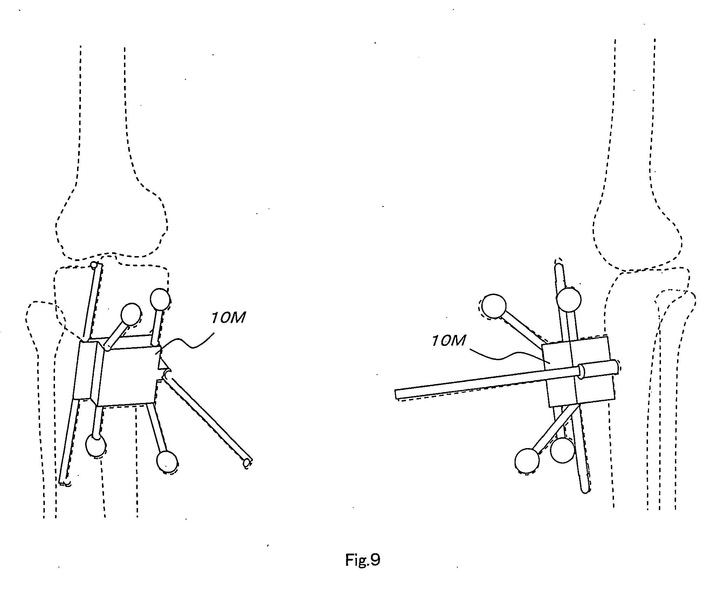

The projected images of the marker as three-dimensional CAD data 10D in Fig. 6 on the plane PS are the left images 10M (CAD images) in Figs. 8 and 9. On the other hand, the projected images of the marker as three-dimensional CAD data 10D in Fig. 7 on the plane PL are the right images 10M (CAD images) in Figs. 8 and 9.
Here, Fig. 8 shows that superimposition of the CAD image 10M on the marker image 10S projected in the radiograph is under way and the marker image 10S and the CAD image 10M are not overlapping each other yet. On the other hand, Fig. 9 shows that the marker image 10S and the CAD image 10M are almost overlapping each other.

Whether or not the CAD image 10M of the marker as three-dimensional CAD data 10D on the plane PS, PL is overlapping the marker image 10S projected in the radiograph is decided according as whether or not the parts corresponding to the ball-like pointing members 16-1 to 16-4 at the tips of the pins 14-1 to 14-4 in the CAD image 10M are overlapping those in the marker image 10S (Fig. 4: Step S4).
In order to facilitate this decision, the parts corresponding to the ball-like pointing members 16-1 to 16-4 (four balls) in the marker as three-dimensional CAD data 10D and the CAD image 10M on the plane PS, PL have different colors.

Here, the temporary center of X rays is set at a position away by a given distance from the center of the radiograph plane vertically. The "given distance" is an initial value entered by the doctor or operator through the input device 114.
The distance between the temporary The source of X-ray and the marker as three-dimensional CAD data 10D, which is not shown in Figs. 6 and 7, can be freely changed; however, at Step S3 or when the CAD image 10M is superimposed on the marker image 10S projected in the radiograph, the distance between the temporary The source of X-ray and the marker as three-dimensional CAD data 10D (not shown) is first set at a fixed value (initial value entered by the doctor or operator through the input device 114) and operation is done to make superimpositions of three of the four ball-like pointing members 16-1 to 16-4. In this stage, superimposition of the remaining ball-like pointing member is almost achieved but not perfect.
Therefore, in order to make superimposition of the remaining ball-like pointing member perfect, an adjustment is made of the distance between the temporary The source of X-ray and the marker as three-dimensional CAD data 10D (not shown).
The marker shown in Fig. 1 is provided with four pins 14-1 to 14-4 and four pointing members 16-1 to 16-4 in order to permit such operation.

As mentioned earlier in connection with Fig. 1, the pins 14-1 to 14-4 and the ball-like pointing members 16-1 to 16-4 are arranged so as not to overlap each other when the marker 10 is observed from the "frontal direction" (vertical direction or direction of arrow S in Fig. 1) and a direction somewhat angled to it. In addition, they are also arranged so as not to overlap each other when the marker 10 is observed from the "lateral direction" (horizontal direction or direction of arrow L in Fig. 1) and a direction somewhat angled to it.
Therefore, the pins 14-1 to 14-4 and the pointing members 16-1 to 16-4 do not overlap each other completely while the process shown in Figs. 5 to 9 is being carried out.

If the four parts corresponding to the ball-like pointing members 16-1 to 16-4 in the marker image 10S all overlap those in the CAD image 10M (Yes at Step S4 in Fig. 4: Fig. 9), this means that the three-dimensional position relation among three elements, the The source of X-ray , radiograph plane and three-dimensional CAD data of the marker 10, is identified. At this time, the position of the The source of X-ray is no longer temporary but identified as the position at the time the radiograph was taken (original position). Then the sequence proceeds to Step S5.
If the four parts corresponding to the pointing members 16-1 to 16-4 are not overlapping (No at Step S4 in Fig. 4: Fig. 8), this means that the three-dimensional position relation (relative three-dimensional position) among three elements, the The source of X-ray , radiograph plane and three-dimensional CAD data of the marker, is not identified. If that is the case, the sequence goes back to Step S3 and operation to superimpose the CAD image 10M on the marker image 10S is continued.

At Step S5, for the frontal radiograph (left in Figs. 6, 8 and 9), a decision is made as to whether or not the four parts corresponding to the ball-like pointing members 16-1 to 16-4 in the marker image 10S are all overlapping those in the CAD image 10M.
If the four parts corresponding to the ball-like pointing members 16-1 to 16-4 in the marker image 10S are overlapping those in the CAD image 10M for both the frontal and lateral radiographs, there are two solutions (relative three-dimensional position relation among three elements, i.e., the The source of X-ray used for taking the radiographs, radiograph plane and three-dimensional CAD data). Here, one solution must be selected. At this time, the doctor or operator looks at the display 112 and selects the solution in which the pins 14-5 to 14-7 of the marker (pins having no ball-like pointing member at their tips) overlap.

If the parts corresponding to the ball-like pointing members 16-1 to 16-4 and pins 14-5 to 14-7 in the marker image 10S all overlap those in the CAD image 10M for both the frontal and lateral radiographs (Yes at Step S5), the sequence proceeds to Step S6.
If the parts corresponding to the ball-like pointing members 16-1 to 16-4 and pins 14-5 to 14-7 in the marker image 10S do not all overlap those in the CAD image 10M for either of the frontal and lateral radiographs (No at Step S5), the sequence goes back to Step 3 and the above process is continued.

At Step 6, the frontal three-element relation determining block 126 determines the three-dimensional relative position relation among three elements, i.e., the frontal The source of X-ray, plane belonging to the frontal radiograph, and three-dimensional CAD data 10D (relation among frontal three elements).
At the same time, the lateral three-element relation determining block 128 determines the three-dimensional relative position relation among three elements, i.e., the lateral The source of X-ray, plane belonging to the lateral radiograph, and three-dimensional CAD data 10D (relation among lateral three elements).

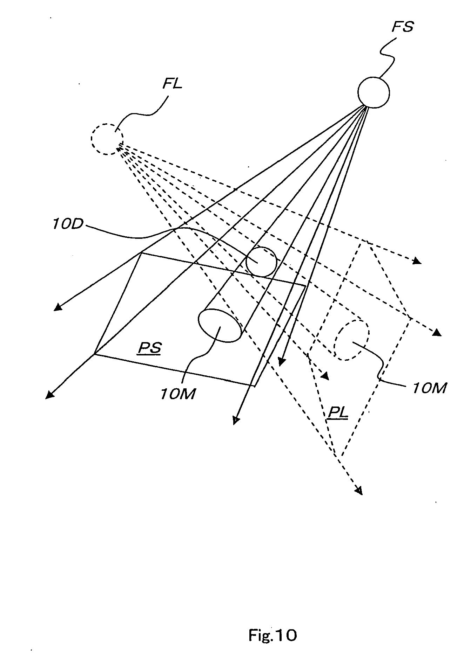

In Fig. 10, the relation among frontal three elements, i.e., the The source of X-ray used for taking the frontal radiograph (frontal The source of X-ray) FS, plane PS belonging to the frontal radiograph, and three-dimensional CAD data 10D, is indicated by solid line.
Also, the relation among lateral three elements, i.e., the The source of X-ray used for taking the lateral radiograph (lateral The source of X-ray) FL, plane PL belonging to the lateral radiograph, and three-dimensional CAD data 10D, is indicated by dotted line.
Thus, Fig. 10 shows the relativethree-dimensionalposition relation between the relation among frontal three elements (frontal three-element relation) and the relation among lateral three elements (lateral three-element relation).
The CAD image in the frontal radiograph and the CAD image in the lateral radiograph are both designated by reference numeral 10M.

In Fig. 10, in the relation among frontal three elements as indicated by solid line, a polyhedral cone (expressed by a conical shape in solid line in Fig. 10 for simple illustration) is defined where the cone has a sectional profile corresponding to the image of the three-dimensional CAD data 10D projected on the plane PS belonging to the frontal radiograph. In the relation among lateral three elements as indicated by dotted line, a polyhedral cone (expressed by a conical shape in dotted line in Fig. 10 for simple illustration) is defined where the cone has a sectional profile corresponding to the image of the three-dimensional CAD data 10D projected on the plane PL belonging to the lateral radiograph.
Here, the marker three-dimensional CAD data 10D is the same in both the "relation among frontal three elements" and the "relation among lateral three elements". Based on this, the relative position relation determining block 130 determines the three-dimensional position relation between the frontal three-element relation and the lateral three-element relation (Step S7).

Once the relative three-dimensional position relation between the frontal three-element relation and the lateral three-element relation has been determined, the relative three-dimensional position relation between the above two polyhedral cones (two conical shapes in Fig. 10) is determined. In that case, when the three-dimensional CAD data 10D is rotated, the rotated image of the three-dimensional CAD data 10D is projected on the plane PL belonging to the frontal radiograph and the plane PL belonging to the lateral radiograph.

Once the relative three-dimensional position relation between the frontal three-element relation and the lateral three-element relation has been determined, namely the relative three-dimensional position relation between the above two polyhedral cones (two conical shapes in Fig. 10) has been determined, the relative three-dimensional position relation among the frontal The source of X-ray FS, plane PS belonging to the frontal radiograph, lateral The source of X-ray FL, plane PL belonging to the lateral radiograph, and three-dimensional CAD data 10D is determined. For this reason, in this description, the "three-dimensional position relation between the frontal three-element relation and the lateral three-element relation" is sometimes referred to as the "relative three-dimensional position relation among the frontal The source of X-ray FS, plane PS belonging to the frontal radiograph, lateral The source of X-ray FL, plane PL belonging to the lateral radiograph, and three-dimensional CAD data 10D."

Once the three-dimensional position relation between the frontal three-element relation and the lateral three-element relation has been determined, when the CAD implant data is projected in the frontal radiograph and lateral radiograph as will be stated later, if the projected image of the implant as three-dimensional CAD data in one radiograph (for example, the frontal radiograph) is moved, the projected image of the implant as three-dimensional CAD data in the other (for example, the lateral radiograph) is also moved in conjunction therewith because the relative three-dimensional position relation among five elements, i.e., the frontal The source of X-ray FS, plane PS belonging to the frontal radiograph, lateral The source of X-ray FL, plane PL belonging to the lateral radiograph, and three-dimensional CAD data 10D has been determined.

The three-dimensional position relation between the frontal three-element relation and the lateral three-element relation is determined using a software technique called "The software 'PhotoModeler'". When the three-dimensional position relation is determined using "The software 'PhotoModeler'", three-dimensional templating is possible even when the frontal radiograph plane and the lateral radiograph plane are not exactly perpendicular (90 degrees) to each other.

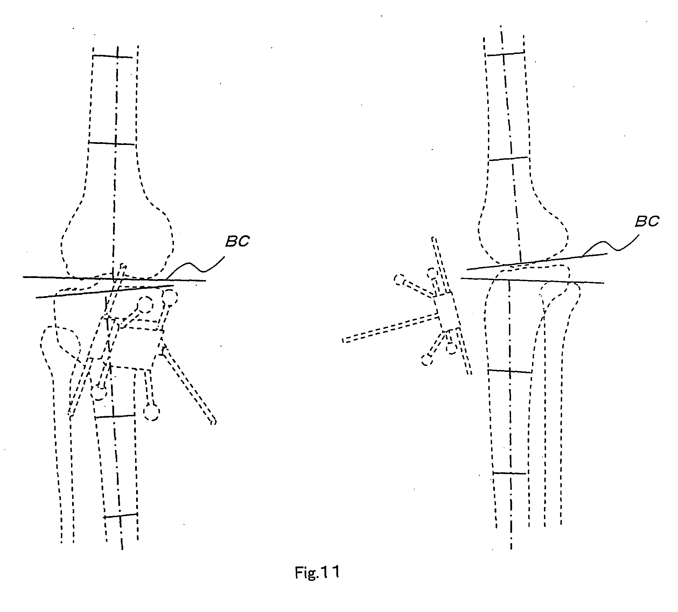

After determination of the three-dimensional position relation between the frontal three-element relation and the lateral three-element relation (after completion of Step S7 in Fig. 4), as illustrated in Fig. 11, the bone axis (functional axis, anatomical axis) and a line perpendicular to the bone axis in the frontal radiograph and lateral radiograph are determined (Step S8 in Fig. 4). This determination can be made by the doctor or operator's manual entry or automatically by specifying four points on the bone cortex as desired.
Since the bone resection plane of the joint is to be perpendicular to the bone axis, the bone axis and a line perpendicular line to it which appear in the radiograph can be used as a guide for determination of the bone resection plane.

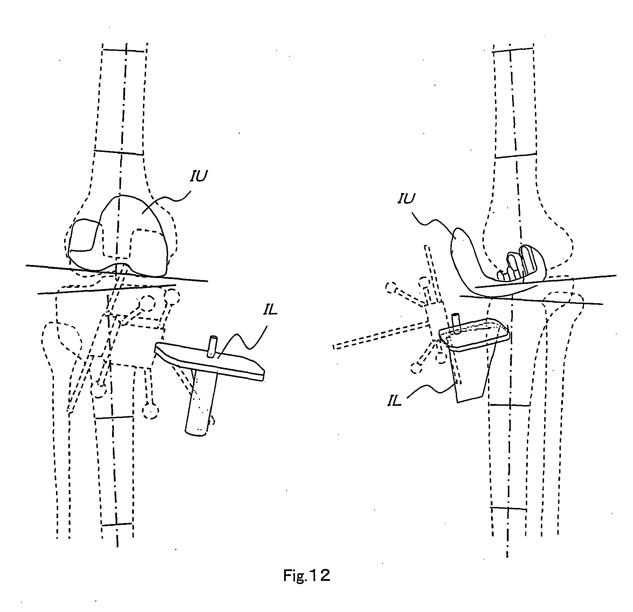

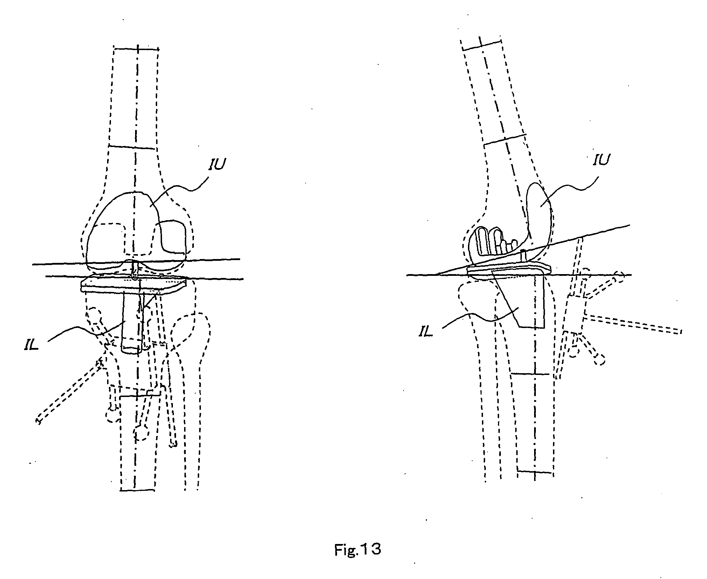

While the three-dimensional position relation between the frontal three-element relation and the lateral three-element relation has been determined, the projected images of the implant as three-dimensional CAD data (implant CAD images IU and IL) are shown in the frontal radiograph and lateral radiograph as illustrated in Figs. 12 and 13.
The doctor or operator adjusts the positions of the shown implant CAD images IU and IL and moves them to an adequate position on the joint in the radiograph (Step S9 in Fig. 4).

Here, for example, in case of a knee joint, the implant CAD image IU is an image of the implant on the femur side as three-dimensional CAD data which is projected on the radiograph plane by the The source of X-ray. Also, for example, in case of a knee joint, the implant CAD image IL is an image of the implant on the tibia and fibula side as three-dimensional CAD data which is projected on the radiograph plane by the The source of X-ray.
Furthermore, Fig. 12 shows a stage in which the size and position of the implant CAD images IU and IL are being adjusted and Fig. 13 shows a stage in which the size and position of the implant CAD images IU and IL are determined.
The implant CAD images IU and IL can be adjusted to a position perpendicular to the bone axis by operation of a means (not shown) .

When the implant CAD images IU and IL are shown in the frontal radiograph and lateral radiograph and the shown implant CAD images IU and IL are moved to an adequate position of the joint in the radiographs (Step S9 in Fig. 4), the three-dimensional implant CAD data stored in the storage block 122, the three-dimensional position relation between the frontal three-element relation and the lateral three-element relation as determined by the relative position relation determining block 130, and operation data entered by the doctor or operator through the input device 114 are sent to the implant position adjusting block 132 (Fig. 3).

Here, in the frontal radiograph and lateral radiograph shown in Figs. 12 and 13, if the implant CAD image IU, for example, in the frontal radiograph is moved, the implant CAD image IU in the lateral radiograph is also moved (in conjunction with the movement in the frontal radiograph).
Furthermore, if an implant CAD image IU of a different size implant is shown in the frontal radiograph, an implant CAD image IU of the different size implant is shown in the lateral radiograph.
With the above operation, the adequate size and three-dimensional position of the implant are determined at Step S9.

In the conventional technique in which only two-dimensional templating can be performed, templating for the frontal radiograph and templating for the lateral radiograph had to be done separately. Since three-dimensional discrepancy in implant position between the frontal and lateral radiographs often occurred, templating was inaccurate.
By contrast, in the embodiment shown here, at Step S9 in Fig. 4 which is carried out while the three-dimensional position relation between the frontal three-element relation and the lateral three-element relation has been determined, as the implant size and position in one radiograph are adjusted, the size and position in the other radiograph are also adjusted in conjunction therewith.

Once the adequate implant size and position have been determined (Yes at Step S10), the sequence proceeds to Step S11.
If the adequate implant size and position have not been determined (No at Step S10), the sequence goes back to Step S9 and the process of determining the adequate size and position of the implant CAD images IU and IL is continued.

At Step S11, the implant CAD image data IU and IL placed in an adequate position of the joint in the frontal radiograph and lateral radiograph are displayed on the display 112 as illustrated in Fig. 13 and also the bone axis and a line perpendicular to it are displayed. In other words, the display 112 shows an implant of an adequate size placed in an adequate position.
At this time, a simulation of a postoperative radiograph as a target which the doctor and operator should achieve is displayed on the display 112.

At the time of completion of Step S11, the adequate implant size and the exact bone resection plane have been determined and templating is finished.
The result of templating is used to determine the bone resection plane in artificial joint replacement.

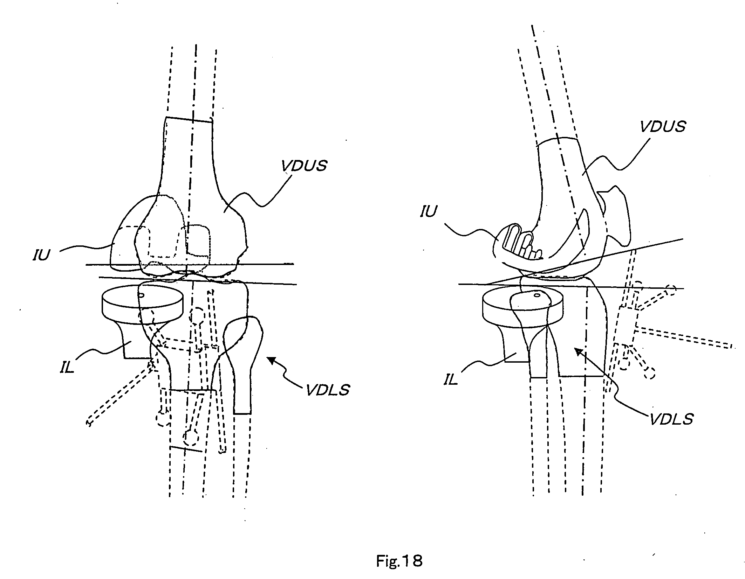

Next, referring to Figs. 14 to 19, a second embodiment of the assisting method for artificial joint replacement and assisting system for artificial joint replacement will be described.
In the first embodiment shown in Figs. 3 to 13, it is difficult to accurately adjust the position of the implant around the bone axis (position in the direction indicated by arrow R in Fig. 19 (c)), the position of so-called the rotation of the implant.
The second embodiment shown in Figs. 14 to 19 is designed so that the implant position relating to such "rotation" can be adjusted adequately.

Fig. 14 shows the system configuration according to the second embodiment.
Referring to Fig. 14, points which are different from the first embodiment shown in Fig. 3 are explained below.
In Fig. 14, the system according to the second embodiment, the whole of which is designated by reference numeral 200, includes a system main unit 210 composed of a computer, a display 112, an input device 114, and X-ray equipment 116 and a CT scanner 212.

The configuration of the system 200 in Fig. 14 according to the second embodiment is the same as that of the system 100 in Fig. 3 according to the first embodiment. Specifically, in Fig. 14 the system main unit 210 includes an input interface 120, a storage block 122 as a database, a marker CAD position adjusting block 124, a frontal three-element relation determining block 126 and a lateral three-element relation determining block 128, a relative position relation determining block 130, an implant position adjusting block 132, a bone axis determining block 134, and an output interface 136.
The system main unit 210 in the second embodiment shown in Fig. 14 further includes a volume data position adjusting block 214 and a volume data creating block 216.

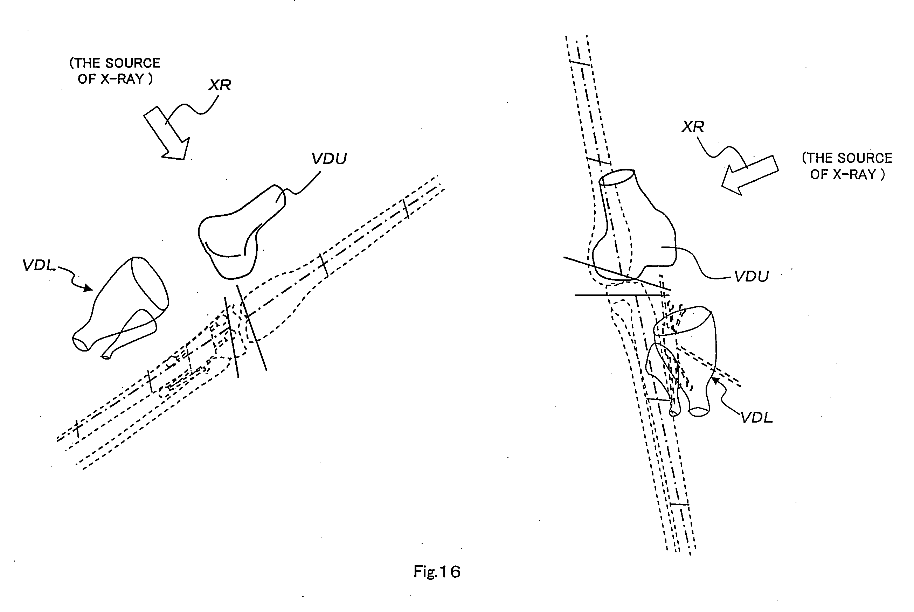

Three-dimensional volume data created in the volume data creating block 216 (three-dimensional volume data is designated by reference symbol VD in Fig. 16) is sent to the volume data position adjusting block 214 through line L32. At the same time, operation information entered by the doctor or operator in charge of the artificial joint replacement assisting operation through the input device 114 (operation data) is sent to the volume data position adjusting block 214 through line L2, the input interface 120, line L5 and line 33. Further, the three-dimensional relative position relation (three-dimensional position relation between the frontal three-element relation and the lateral three-element relation) is sent to the volume data position adjusting block 214 through line L34.

The volume data position adjusting block 214 is designed to superimpose a projected image of three-dimensional volume data on the radiograph of the joint in the frontal and lateral radiographs based on operation data.
In the volume data position adjusting block 214, operation to superimpose the projected image of volume data on the radiograph of the joint is performed. The projected image of volume data superimposed on the radiograph of the joint is displayed on the display 112 through lines L35 and L37, the output interface 136 and line L38.

The patient' s joint to undergo artificial joint replacement is scanned by the CT scanner 212 and the scan data from the CT scanner 212 is sent to the storage block 122 through line L39, the input interface 120 and line L40 and stored in it. The CT scanner 212' s scan data stored in the storage block 122 is sent to the volume data creating block 216 through line L31.
The volume data creating block 216 creates three-dimensional volume data including three-dimensional information from the CT scanner 212's scan data using a known software technique.

The processing sequence of assisting artificial joint replacement using the assisting system for artificial joint replacement 200 shown in Fig. 14, specifically the processing sequence of performing templating, is explained below referring to Figs. 15 to 19.
In Fig. 15, at the steps marked "S1∼S8" (the individual steps are not marked here), the same steps as steps S1 to S8 in Fig. 4 are carried out. Specifically, the marker 10 is fixed in the vicinity of the joint (in case of a knee joint, on the front of the tibia) and a frontal radiograph and a lateral radiograph are taken (Step S1); the frontal radiograph and lateral radiograph in which an image of the marker 10 is shown is entered into the system main unit as data (Step S2) ; the marker CAD image 10M is superimposed on the image 10S of the marker 10 projected in each of the frontal and lateral radiographs (Steps S3 to S5); the relation among frontal three elements and the relation among lateral three elements are determined (Step S6); the relative position relation between the frontal three-element relation and the lateral three-element relation (Step S7); and the bone axis is marked in the radiographs (Step S8).

In the second embodiment in Fig. 15, at Step S11 the patient' s joint to undergo artificial joint replacement is scanned by the CT scanner 212. At Step S2, the data (in DICOM data format) obtained by scanning with the CT scanner 212 is sent to the storage block 122 of the system main unit 210 through line L39, the input interface 120 and line L40 and stored in it.

The CT scanner 212' s scan data stored in the storage block 122 (data obtained by scanning the patient's joint to undergo artificial joint replacement by the CT scanner 212) is sent to the volume data creating block 216 and three-dimensional volume data including three-dimensional information on the patient' joint is created there. A known software technique is used to create such three-dimensional volume data.
In creation of three-dimensional volume data, volume data on the upper and lower bones which surround the joint (in case of a knee joint, femur, and tibia and fibula) is divided into two kinds of volume data (reference symbols VDU, VDL in Fig. 16). In dividing the data on the bones surrounding the joint, the doctor or operator specifies a boundary (namely the joint) (in the volume data to be divided) on the joint slice data displayed on the display 112. The divided volume data is converted into three-dimensional CAD data so that an image projected on a radiograph plane (volume data image) can be created with the The source of X-ray

Next, as illustrated on the left in Fig. 16, regarding the relation among frontal three elements, three-dimensional volume data VDU and VDL are placed between the The source of X-ray (not clearly shown in Fig. 16) and the radiograph. Also, as illustrated on the right in Fig. 16, regarding the relation among lateral three elements, three-dimensional volume data VDU and VDL are placed in the same way (Step S12). Then, the three-dimensional volume data VDU and VDL are irradiated with X rays XR from the The source of X-ray to project images VDUS and VDLS (volume data images) of the three-dimensional volume data VDU and VDL in the relation among frontal three elements (left in Fig. 6) and the relation among lateral three elements (right in Fig. 16).

At this stage, the doctor or operator adjusts the positions of the volume data images VDUS and VDLS. Specifically, the volume data position adjusting block 214 (Fig. 14) adjusts the three-dimensional positions of the three-dimensional volume data VDU and VDL according to information entered by the doctor or operator (operation information for adjusting the positions of the volume data images VDUS and VDLS) so that the volume data images VDUS and VDLS are superimposed on the image of the joint in the frontal radiograph (left in Fig. 16) and lateral radiograph (right in Fig. 16).

Since the three-dimensional position relation between the frontal three-element relation and the lateral three-element relation has been determined at Step S8, as the positions of the volume data images VDUS and VDLS in one of the radiographs are moved and adjusted, the volume data images VDUS and VDLS in the other radiograph are also moved in conjunction therewith and such movement is displayed. In other words, the process of adjusting the volume data images VDUS and VDLS to superimpose them on the image of the joint in the frontal radiograph (left in Fig. 16) is carried out three-dimensionally in conjunction with the process of adjusting the volume data images VDUS and VDLS to superimpose them on the image of the joint in the lateral radiograph (right in Fig. 16).

At Step S13, a decision is made as to whether or not the volume data images VDUS and VDLS are superimposed on the image of the joint in the frontal radiograph (left in Fig. 16) and that in the lateral radiograph (right in Fig. 16)
The doctor or operator makes this decision, looking at the images on the display 112. Alternatively, the system may make this decision (under automatic control).

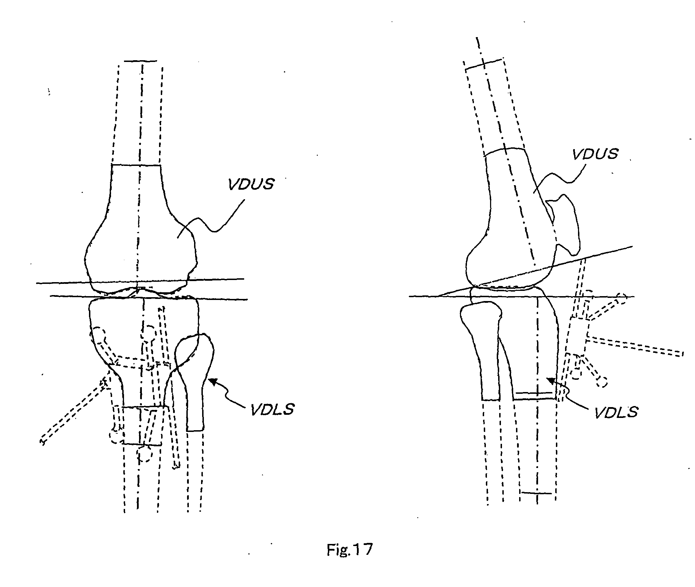

If the volume data images VDUS and VDLS are superimposed on the image of the joint in the radiographs (Yes at Step S13: Fig. 17), the sequence proceeds to Step S14.
If not (No at Step S13), the process of adjusting the volume data images VDUS and VDLS to superimpose them on the image of the joint in the frontal radiograph (left in Fig. 16) and that in the lateral radiograph (right in Fig. 6) (Step S12) is continued (loop for "No" at Step S13).

At Step S14, the display 112 shows that the volume data images VDUS and VDLS are superimposed on the image of the joint in the frontal radiograph (left in Fig. 16) and that in the lateral radiograph (right in Fig. 6) (Fig. 17).
The doctor or operator adjusts the position of the three-dimensional CAD data of the implant, looking at the images on the display 112. Particularly, he/she adjusts CAD the rotation of the implant with respect to the bone axis.

Again at Step S14, since the three-dimensional position relation between the frontal three-element relation and the lateral three-element relation has been determined, as the position of the implant CAD image in one of the radiographs is moved and adjusted, the implant CAD image in the other radiograph is also moved in conjunction therewith.
In the second embodiment, position relations which could not be checked in the first embodiment, namely three-dimensional relations of the implant CAD image with respect to the bones, can be determined by using three-dimensional volume data. An example of position relation which can be determined only in the second embodiment is a position relation on a plane perpendicular to the bone axis as illustrated in Fig. 19(c).

Fig. 19 (c) is a view of the resection shown in Figs. 19 (a) and (b) on a plane BC perpendicular to the bone axis from the direction of arrow (c) (the resection status is displayed on the display 112).
Since the three-dimensional volume data VDU consists of scan data from the CT scanner 212, the status of the resection made on the plane BC perpendicular to the bone axis can be represented. Fig. 19(c) also shows a cross section of the three-dimensional CAD data of the implant (three-dimensional CAD data VDU of the femur side implant) on the plane BC.

In the first embodiment, the position in the direction of arrow R in Fig. 19(c), so-called rotation, cannot be displayed on the display 112 and the rotational position of the three-dimensional CAD data of the implant cannot be adjusted.
By contrast, in the second embodiment, as explained above, it can be displayed on the display 112 as illustrated in Fig. 19(c), so regarding the position in the direction of arrow R in Fig. 19(c), so-called rotation, the rotational position of the three-dimensional CAD data of the implant can be adjusted.
Thus, the position of the implant can be determined with higher accuracy than in the first embodiment shown in Figs. 3 to 13.

At Step S14, the adequate implant size can be determined by using three-dimensional CAD data of different implant sizes. In other words, the adequate size and position (including rotation) of the implant can be adjusted three-dimensionally at Step S14.
At Step S15, a decision is made as to whether the adequate size and position of the implant have been determined and if the adequate position has been determined (Yes at Step S15), the sequence proceeds to Step S16.
If the adequate size and position of the implant have not been determined (No at Step S15), the process of adjusting the adequate size and position (including rotation) of the implant three-dimensionally is continued (loop for "No" at Step S15).

At Step S16, since the adequate size and position of the implant for the patient's joint to undergo artificial joint replacement have been determined, the bone resection line is determined based on this.
As described above, such bone resection line can be determined very accurately regardless of the angle between the frontal radiograph and lateral radiograph. Since the three-dimensional position and size of the implant and the bone resection line can be determined accurately prior to starting artificial joint replacement, the volume of the bone to be resected can also be determined. During an operation for artificial joint replacement, the practitioner can check if the operation is being carried out properly, by comparing the volume of the bone being resected with the expected volume of the resected bone.

The other constituent parts, function and effect of the second embodiment illustrated in Figs. 14 to 19 are the same as those of the first embodiment illustrated in Figs. 3 to 13.

The embodiments illustrated here are illustrative and not restrictive and the above detailed description thereof is not intended to limit the technical scope of the invention.
For instance, although artificial knee joints are shown in the embodiments illustrated here, the invention may be applied to any kind of artificial joint replacement.

### BRIEF DESCRIPTION OF THE DRAWINGS

[Fig. 1] is a perspective view of an assisting device for artificial joint replacement (marker) according to the present invention.
[Fig.2] is a longitudinal sectional view of an assisting device for artificial joint replacement.
[Fig. 3] is a block diagram showing an assisting system for artificial joint replacement according to a first embodiment of the present invention.
[Fig. 4] is a flowchart showing control operation of the assisting system for artificial joint replacement according to the first embodiment of the present invention (or an assisting method for artificial joint replacement according to the first embodiment of the present invention).
[Fig. 5] is a view illustrating radiographs loaded in the system main unit.
[Fig. 6] is a view illustrating a three-dimensional position relation among an The source of X-ray , three-dimensional CAD data of the marker and a frontal radiograph plane.
[Fig. 7] is a view illustrating a three-dimensional position relation among an The source of X-ray , three-dimensional CAD data of the marker and a lateral radiograph plane.
[Fig. 8] is a view illustrating the process of superimposing a proj ectedmarker image as three-dimensional CAD data on a marker image projected in the radiograph.
[Fig. 9] is a view illustrating the projected marker image as three-dimensional CAD data superimposed on the marker image projected in the radiograph.
[Fig. 10] is a diagram illustrating the dimensional relative position relation among the frontal The source of X-ray, plane belonging to the frontal radiograph, lateral The source of X-ray, plane belonging to the lateral radiograph and three-dimensional CAD data.
[Fig. 11] is a view illustrating a bone axis and a line perpendicular to the bone axis which are shown in the radiograph. [Fig. 12] is an explanatory view showing the process of adjusting the projected implant image as three-dimensional CAD data to place it in an adequate position in the radiograph.
[Fig. 13] is a view illustrating the projected implant image as three-dimensional CAD data imposed in an adequate position in the radiograph.
[Fig. 14] is a block diagram showing an assisting system for artificial joint replacement according to a second embodiment. [Fig. 15] is a flowchart showing control operation of an assisting system for artificial joint replacement according to a second embodiment (or an assisting method for artificial joint replacement according to the second embodiment).
[Fig. 16] is a view illustrating a three-dimensional position relation among an The source of X-ray, three-dimensional volume data and a radiograph.
[Fig. 17] is a view illustrating a projected implant image as three-dimensional volume data superimposed in an adequate position of the radiograph.
[Fig. 18] is a view illustrating the process of superimposing the projected image as three-dimensional CAD data in an adequate position in the radiograph where the projected volume data image is superimposed.
[Fig. 19] is a view illustrating the projected implant image as three-dimensional CAD data superimposed in an adequate position in the radiograph where the projected volume data image is superimposed.

### DESCRIPTION OF THE REFERENCE NUMERALS AND SYMBOLS

10...Marker (roentgen marker)
10D...Marker three-dimensional CAD data
10M...Projected marker image as three-dimensional CAD data in a radiograph
10S...Image projected in a radiograph
12...Marker main unit
14-1 to 14-7...Pins
16-1 to 16-4...Pointing members
18-1, 18-2...Attachment flanges
100, 200...Artificial join replacement assisting systems
110, 210...System main units
112...Display (display unit)
114...Input means
116...X-ray equipment
120...Input interface
122...Storage block (database)
124...Marker CAD position adjusting block
126...Frontal three-element relation determining block
128...Lateral three-element relation determining block
130...Relative position relation determining block
132...Implant position adjusting block
134...Bone axis determining block
136...Output interface
212...CT scanner
214...Volume data position adjusting block
216...Volume data creating block
IU...Three-dimensional CAD data of the implant on the femur side
IL...Three-dimensional CAD data of the implant on the tibia side
VDU, VDL...Three-dimensional volume data
XR...X rays
F...The source of X-ray
FS...Frontal The source of X-ray
FL...Lateral The source of X-ray

## Claims

1. An assisting device for artificial joint replacement comprising:
a main body (12);
four pins (14-1 to 14-4) attached to outer edges of the main body (12);
horizontal pins (14-5, 14-6) attached to the main body through a first attachment flange (18-1) and extending horizontally (direction of arrow L); and
a vertical pin (14-7) attached to the main body through a second attachment flange (18-2) and extending vertically (direction of arrow S),
the four pins (14-1 to 14-4) having ball-like pointing members (16-1 to 16-4) at their tips respectively,
wherein the four pins (14-1 to 14-4) and the pointing members (16-1 to 16-4) are so arranged as:
not to overlap each other when seen from above and a vertical direction (direction of arrow S) and a direction angled to the vertical direction; and
not to overlap each other when seen from a horizontal direction (direction of arrow L) and a direction angled to the horizontal direction (direction of arrow L).

2. A computer-based assisting method for artificial joint replacement comprising the steps of:
placing the assisting device for artificial joint replacement (10) as described in Claim 1 in the vicinity of a patient's joint to undergo artificial joint replacement and taking radiographs from two directions (S1);
putting three-dimensional CAD data (10D) of the assisting device for artificial joint replacement (10) between an The source of X-ray and a radiograph, displaying it on a display (112), and superimposing a three-dimensional CAD data image (10M) of the assisting device for artificial joint replacement projected (10) projected in the radiograph, on a image (10S) of the assisting device for artificial joint replacement projected in the radiograph (S3 to S5);
determining a relative three-dimensional position relation among an The source of X-ray for a frontal radiograph, three-dimensional CAD data (10D) of the assisting device for artificial joint replacement (10) and a frontal radiograph plane and a relative three-dimensional position relation among an The source of X-ray for a lateral radiograph, three-dimensional CAD data (10D) of the assisting device for artificial joint replacement (10) and a lateral radiograph plane (S6);
determining, from the relative three-dimensional position relation for the frontal radiograph and the relative three-dimensional position relation for the lateral radiograph as determined at the preceding step (S6), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (S7); and
superimposing projected implant images (IU, IL) as there-dimensional CAD data in an adequate position of an image of the joint in a radiograph in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined, and displaying it on the display (112) (S9, S10).

3. A computer-based assisting method for artificial joint replacement comprising the steps of:
placing the assisting device for artificial joint replacement (10) as described in Claim 1 in the vicinity of a patient's joint to undergo artificial joint replacement and taking radiographs from two directions (S1);
scanning the patient's joint to undergo artificial joint replacement by CT equipment (212) (S1);
putting three-dimensional CAD data (10D) of the assisting device for artificial j oint replacement (10) between an The source of X-ray and a radiograph, displaying it on a display (112), and superimposing a three-dimensional CAD data image (10M) of the assisting device for artificial joint replacement (10) projected in the radiograph, on an image (10S) of the assisting device for artificial joint replacement (10) projected in the radiograph (S3 to S5);
determining a relative three-dimensional position relation among an The source of X-ray for a frontal radiograph, three-dimensional CAD data (10D) of the assisting device for artificial joint replacement (10) and a frontal radiograph plane and a relative three-dimensional position relation among an The source of X-ray for a lateral radiograph, three-dimensional CAD data (10D) of the assisting device for artificial joint replacement (10) and a lateral radiograph plane (S6);
determining, from the relative three-dimensional position relation for the frontal radiograph and the relative three-dimensional position relation for the lateral radiograph as determined at the preceding step (S6), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (S7);
creating, from data obtained by scanning the patient's joint to undergo artificial joint replacement by CT equipment (212), three-dimensional volume data (VDU, VDL) including three-dimensional data of the patient's joint (S11);
putting the three-dimensional volume data (VDU, VDL) between an The source of X-ray and a radiograph and superimposing three-dimensional volume data images (VDUS, VDLS) projected in the radiograph, on a joint part shown in the radiograph (S12, S13); and
adjusting rotational (R) position of the implant as three-dimensional CAD data to superimpose implant images (IU, IL) as there-dimensional CAD data in an adequate position of the joint in a radiograph in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) has been determined and the three-dimensional volume data images (VDUS, VDLS) are superimposed on the joint part, and displaying it on the display (112) (S14, S15).

4. An assisting system for artificial joint replacement including a system main unit (110) which receives radiographs taken from two directions with the assisting device for artificial joint replacement (10) as described in Claim 1 placed in the vicinity of a patient's joint to undergo artificial joint replacement, and a display (112),
the system main unit (110) comprising:
a device (124) designed to put three-dimensional CAD data (10D) of the assisting device for artificial joint replacement (10) between an The source of X-ray and a radiograph and superimpose a three-dimensional CAD data image (10M) of the assisting device for artificial joint replacement (10) projected in the radiograph, on an image (10S) of the assisting device for artificial joint replacement (10) projected in the radiograph;
a device (126) designed to determine a relative three-dimensional position relation among an The source of X-ray for a frontal radiograph, three-dimensional CAD data (10D) of the assisting device for artificial joint replacement (10) and a frontal radiograph plane;
a device (128) designed to determine a relative three-dimensional position relation among an The source of X-ray for a lateral radiograph, three-dimensional CAD data (10D) of the assisting device for artificial joint replacement (10) and a lateral radiograph plane;
a device (130) designed to determine, from the relative three-dimensional position relation for the frontal radiograph and the relative three-dimensional position relation for the lateral radiograph, a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D); and
a device (132) designed to superimpose projected implant images (IU, IL) as there-dimensional CAD data in an adequate position of an image of the joint in a radiograph in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined, and display it on the display (112).

5. An assisting system for artificial joint replacement including a system main unit (110) and a display (112),
the system main unit (110) designed to receive radiographs taken from two directions with the assisting device for artificial joint replacement (10) placed in the vicinity of a patient's joint to undergo artificial joint replacement and receive scan data obtained by scanning the patient's joint to undergo artificial joint replacement by CT equipment (212); and
the system main unit (110) comprising:
a device (124) designed to put three-dimensional CAD data (10D) of the assisting device for artificial joint replacement (10) between an The source of X-ray and a radiograph and superimpose a three-dimensional CAD data image (10M) of the assisting device for artificial joint replacement projected (10) in the radiograph, on a projected image (10S) of the assisting device for artificial joint replacement in the radiograph;
a device (126) designed to determine a relative three-dimensional position relation among an The source of X-ray for a frontal radiograph, three-dimensional CAD data (10D) of the assisting device for artificial joint replacement (10) and a frontal radiograph plane;
a device (128) designed to determine a relative three-dimensional position relation among an The source of X-ray for a lateral radiograph, three-dimensional CAD data (10D) of the assisting device for artificial joint replacement (10) and a lateral radiograph plane;
a device (130) designed to determine, from the relative three-dimensional position relation for the frontal radiograph and the relative three-dimensional position relation for the lateral radiograph), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D);
a device (216) designed to create, from data obtained by scanning the patient's joint to undergo artificial joint replacement by CT equipment (212), three-dimensional volume data (VDU, VDL) including three-dimensional data of the patient's joint;
a device (214) designed to put the three-dimensional volume data (VDU, VDL) between an The source of X-ray and a radiograph, display it on the display (112) and superimpose three-dimensional volume data images (VDUS, VDLS) projected on the radiographplane, on a joint part shown in the radiograph; and
a device (implant position adjusting block 132) designed to adjust rotational (R) position of the implant as three-dimensional CAD data to superimpose the implant images (IU, IL) as there-dimensional CAD data in an adequate position of the joint in a radiograph in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) has been determined and the three-dimensional volume data images (VDUS, VDLS) are superimposed on the joint part, and display it on the display (112).

6. A computer program designed to perform the steps of:
inputting radiographs taken from two directions with the assisting device for artificial joint replacement (10) as described in Claim 1 placed in the vicinity of a patient's joint to undergo artificial joint replacement(S2);
putting three-dimensional CAD data (10D) of the assisting device for artificial joint replacement (10) between an The source of X-ray and a radiograph, and displaying it on a display (112) (S3), and superimposing a three-dimensional CAD data image (10M) of the assisting device for artificial joint replacement (10) projected in the radiograph, on an image (10S) of the assisting device for artificial joint replacement projected in the radiograph in response to operation data entered through an input device (114) (S3 to S5);
determining a relative three-dimensional position relation among an The source of X-ray for a frontal radiograph, three-dimensional CAD data (10D) of the assisting device for artificial joint replacement (10) and a frontal radiograph plane and a relative three-dimensional position relation among an The source of X-ray for a lateral radiograph, three-dimensional CAD data (10D) of the assisting device for artificial joint replacement (10) and a lateral radiograph plane (S6);
determining, from the relative three-dimensional position relation for the frontal radiograph and the relative three-dimensional position relation for the lateral radiograph as determined at the preceding step (S6), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (S7); and
superimposing projected implant images (IU, IL) as there-dimensional CAD data in an adequate position of an image of the joint in a radiograph in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (three-dimensional position relation between the frontal three-element relation and the lateral three-element relation) has been determined, and displaying it on the display (112) (S9, S10) .

7. A computer program designed to perform the steps of:
inputting radiophotographs taken from two directions with the assisting device for artificial joint replacement (10) as described in Claim 1 placed in the vicinity of a patient's joint to undergo artificial joint replacement and inputting data obtained by scanning the patient's joint to undergo artificial joint replacement by CT equipment (212) (S2);
putting three-dimensional CAD data (10D) of the assisting device for artificial joint replacement (10) between an The source of X-ray and a radiograph, displaying it on a display (112), and superimposing a three-dimensional CAD data image (10M) of the assisting device for artificial joint replacement (10) projected in the radiograph, on an image (10S) of the assisting device for artificial joint replacement (10) projected in the radiograph (S3 to S5);
determining a relative three-dimensional position relation among an The source of X-ray for a frontal radiograph, three-dimensional CAD data (10D) of the assisting device for artificial joint replacement (10) and a frontal radiograph plane and a relative three-dimensional position relation among an The source of X-ray for a lateral radiograph, three-dimensional CAD data (10D) of the assisting device for artificial joint replacement (10) and a lateral radiograph plane (S6);
determining, from the relative three-dimensional position relation for the frontal radiograph and the relative three-dimensional position relation for the lateral radiograph as determined at the preceding step (S6), a three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) (S7);
creating, from data obtained by scanning the patient's joint to undergo artificial joint replacement by CT equipment (212), three-dimensional volume data (VDU, VDL) including three-dimensional data of the patient's joint (S11);
putting the three-dimensional volume data (VDU, VDL) between an The source of X-ray and a radiograph plane and superimposing three-dimensional volume data images (VDUS, VDLS) projected in the radiograph, on a joint part shown in the radiograph (S12, S13); and
adjusting rotational (R) position of the implant as three-dimensional CAD data to superimpose the implant images (IU, IL) as there-dimensional CAD data in an adequate position of the joint in a radiograph in which the three-dimensional relative position relation among the frontal The source of X-ray (FS), plane (PS) belonging to the frontal radiograph, lateral The source of X-ray (FL), plane (PL) belonging to the lateral radiograph, and three-dimensional CAD data (10D) has been determined and the three-dimensional volume data images (VDUS, VDLS) are superimposed on the joint part, and displaying it on the display (112) (S14, S15).
